(19) Europäisches Patentamt
European Patent Office
Office européen des brevets

(11) EP 3 212 201 B1

(12) # EUROPEAN PATENT SPECIFICATION

(45) Date of publication and mention of the grant of the patent:
**19.10.2022 Bulletin 2022/42**

(21) Application number: **15801122.1**

(22) Date of filing: **26.10.2015**

(51) International Patent Classification (IPC):
***A61K 31/7076*** *(2006.01)* ***C07H 19/167*** *(2006.01)*
***A61P 35/00*** *(2006.01)* ***A61P 3/00*** *(2006.01)*
***A61P 19/02*** *(2006.01)* ***A61P 1/00*** *(2006.01)*
***A61P 9/00*** *(2006.01)* ***A61P 27/00*** *(2006.01)*
***A61P 25/28*** *(2006.01)* ***A61P 17/06*** *(2006.01)*

(52) Cooperative Patent Classification (CPC):
**A61K 31/7076; A61P 1/00; A61P 3/00; A61P 9/00; A61P 17/06; A61P 19/02; A61P 25/28; A61P 27/00; A61P 35/00; C07H 19/167**

(86) International application number:
**PCT/EP2015/074741**

(87) International publication number:
**WO 2016/066582 (06.05.2016 Gazette 2016/18)**

(54) **NUCLEOSIDE KINASE INHIBITORS**

NUKLEOSIDKINASEINHIBITOREN

INHIBITEURS DE LA KINASE NUCLÉOSIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2014 EP 14190634**

(43) Date of publication of application:
**06.09.2017 Bulletin 2017/36**

(73) Proprietor: **BCI Pharma**
**34790 Grabels (FR)**

(72) Inventor: **SURLERAUX, Dominique**
**34790 Grabels (FR)**

(74) Representative: **Gevers Patents**
**Intellectual Property House**
**Holidaystraat 5**
**1831 Diegem (BE)**

(56) References cited:
**EP-A2- 0 269 574**
**WO-A1-01/40245**
**WO-A1-96/40705**
**WO-A1-03/104482**
**WO-A1-2004/022572**
**WO-A2-2005/117882**

- **VENKATA P PALLE ET AL: "Affinity and intrinsic efficacy (IE) of 5'-carbamoyl adenosine analogues for the A1 adenosine receptor-efforts towards the discovery of a chronic ventricular rate control agent for the treatment of atrial fibrillation (AF)", BIOORGANIC & MEDICINAL CHEMISTRY LETTERS, vol. 14, no. 2, 1 January 2004 (2004-01-01), pages 535-539, XP055183658, ISSN: 0960-894X, DOI: 10.1016/j.bmcl.2003.09.094**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 212 201 B1

## Description

### Field of the invention

The present invention is in the field of medicinal chemistry and pharmaceuticals.

### Background of the invention

A lot of progress has been made in the understanding of biomolecular pathways and the roles they play in disease is generating a large number of targets for therapeutic inventions. Protein kinases are one class of therapeutic targets.

Kinases play a key role in almost all signal transduction pathways. A kinase is a type of enzyme that transfers phosphate groups from high-energy donor molecules, such as adenosine triphosphate (ATP), to specific substrates, a process referred to as phosphorylation. The phosphorylation state of a substrate, whether it is a protein, lipid, or carbohydrate, can affect its activity, reactivity, and its ability to bind other molecules. Therefore, kinases are critical in metabolism, cell signalling, protein regulation, cellular transport, secretory processes, and many other cellular pathways. Protein kinases play pivotal roles in many diseases, such as cancer, metabolic diseases, rheumatoid arthritis, psoriasis, inflammatory bowel disease, cardiovascular diseases, Vogt-Koyanagi-Harada syndrome (non-infectious blindness), and Alzheimer. Inhibiting kinases, and therefore phosphorylation, can treat these diseases. Therefore, kinase inhibitors can be used as drugs.

During phosphorylation, the phosphate groups are usually added to the serine, threonine or tyrosine amino acid on the protein. Hence, protein kinase inhibitors can be subdivided or characterized by the amino acids whose phosphorylation is inhibited: most kinases act on both serine and threonine, the tyrosine kinases act on tyrosine, and some kinases act on all three amino acids. There are also protein kinases that phosphorylate other amino acids, such as histidine kinases.

There is thus a need for the development of drugs that can interact with selected targets, such as protein kinases. A number of kinase inhibitors have appeared in the scientific literature, and some have entered human clinical trials.

WO 2004/022572 (Alchemia Pty Ltd) discloses classes of biologically active compounds interacting with kinases, and the preparation of these compounds.

EP 0269574 (Nippon Zoki Pharmaceutical Co. Ltd.) discloses adenosine compounds for use in the treatment of hypertension, cerebrovascular disease, cardiopathy or renal insufficiency.

WO 2003/104482 (Metabolic Engineering Laboratories Co., Ltd) discloses a composition for modulating cellular senescence and useful for the treatment of Alzheimer's disease or atherosclerosis, said composition comprising the inhibitor of protein kinase A such as adenosine 3'5'-cyclin phosphorothiolates.

WO 1996/040705 (Gensia Inc.) discloses adenosine kinase inhibitors compounds 149-175, 413-431, and 241-266, for use in the treatment of cardiovascular and cerebrovascular diseases, inflammation, arthritis, and cancer.

Palle et al. (Bioorganic & Medicinal Chemistry Letters 14 (2004) 535-539) discloses A1 adenosine partial or full agonists for use in the treatment of atrial fibrillation.

WO 2001/040245 (CV Therapeutics Inc.) discloses a method of cardioprotection by using adenosine A1 receptor partial or full agonists.

WO 2005/117882 (Incyte Corporation) discloses ligands of C5a receptor, for use in the treatment of diseases associated with metalloprotease activity such as arthritis, cancer, cardiovascular disorders, skin disorders, inflammation or allergic conditions.

Cottam et al. (Journal of Medicinal Chemistry, 1993, Vol. 36, No. 22) discloses adenosine kinase inhibitors with oral antiinflammatory activity, particularly compound 20, for which a proviso is included under the definition of $R^{11}$ and $R^{12}$ in the present patent.

WO 2004/022572 A1 discloses protein kinase inhibitors for use in the treatment of different conditions including leukemia, restenosis, psoriasis or atherosclerosis.

WO 03/104482 A1 discloses a composition for modulating cellular senescence, and thus a composition useful for the treatment of Alzheimer's disease or atherosclerosis, said composition comprising the inhibitor of protein kinase A such as adenosine 3',5'-cyclin phophorothiolates.

WO 96/40705 A1 discloses adenosine kinase inhibitors for use in the treatment of cardiovascular and cerebrovascular diseases, inflammation and other diseases which can be regulated by increasing the local concentration of adenosine such as arthritis and cancer.

Palle et al. (Bioorganic & Medicinal Chemistry Letters, 2004, 14(2), 535-539) discloses adenosine partial or full agonists for use in the treatment of atrial fibrillation and the method for preparation thereof.

WO 01/40245 A1 discloses a method of cardio protection by using adenosine A1 receptor partial or full agonists.

WO 2005/117882 A2 discloses a ligand of C5a receptor for use in the treatment of diseases associated with metalloprotease activity such as arthritis, cancer, cardiovascular disorders, skin disorders, inflammation or allergic conditions.

EP 0269574 A2 discloses adenosine derivatives useful as antihypertensive agents, for example, for the treatments of hypertension and various diseases caused by hypertension, such as cerebrovascular diseases, cardiopathy or renal insufficiency.

**Summary of the invention**

It is an objective of the present invention to provide a method of inhibiting protein kinase activity, whether in vitro, in vivo, or in silico.

It is an objective of the present invention to provide a collection of protein kinase inhibitors, in order to build up a chemical library for testing specific protein kinase inhibition, or for testing inhibition of specific combinations of protein kinases. Each chemical of the library has associated information stored in a database, with information such as the chemical structure, purity, quantity, physicochemical characteristics, and biological activities of each of the compounds.

It is an objective of the present invention to provide a chemical library of candidate compounds for testing specific protein kinase inhibition, useful for the pharmaceutical industry in its (pre-)clinical selection of drug candidates.

It is an objective of the present invention to provide protein kinase inhibitors, which can be used to treat a plurality of diseases.

There is a need for protein kinase inhibitors which may overcome the disadvantages of current protein kinase therapy such as side effects, limited efficacy, the emerging of resistance, and compliance failures.

The present disclosure relates to a method of inhibiting protein kinase activity, as well as inhibitors of protein kinase which exhibit at least one improved property in view of the compounds of the prior art. In particular, the inhibitors of the present invention are interesting in one or more of the following pharmacological related properties, i.e. potency profile, potency against specific kinases, decreased toxicity, decreased cytotoxicity, improved pharmacokinetics, acceptable dosage, processing ease, and the like.

The present invention relates to an *in vitro* method of inhibiting protein kinase activity which comprises contacting a protein kinase with a compound of formula (II), or the N-oxide, pharmaceutically acceptable salt, pharmaceutically acceptable solvate, or stereoisomer thereof,

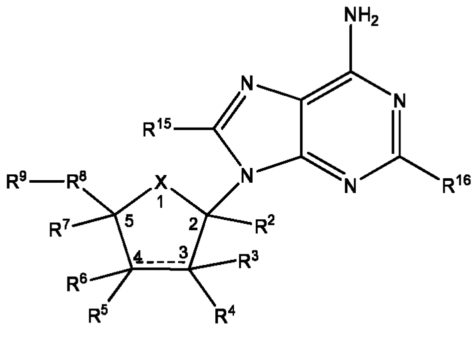

**(II)**

wherein

the dash bond (represented by ------ ) represents an optional double bond; provided that when there is a double bond in the 5-membered ring, the substituents $\mathbf{R^3}$ and $\mathbf{R^6}$ are not present;

X is selected from O and S;

$\mathbf{R^2}$ is selected from hydrogen; Het; aryl; $C_{1-6}$alkyl optionally substituted with halo, hydroxy, or Het; halo$C_{1-6}$alkyl; $C_{2-6}$alkenyl; $C_{2-6}$alkynyl; - C(=O)$NR^{11}R^{12}$; and a cyclo$C_{3-7}$alkyl or Het which is formed with any one of the carbon atoms of the depicted cyclopentane ring except carbon atom 2;

$\mathbf{R^3}$, $\mathbf{R^4}$, $\mathbf{R^5}$, $\mathbf{R^6}$, and $\mathbf{R^7}$ are each independently selected from hydrogen; halo; cyano; oxo; -$NR^{11}R^{12}$; -$OR^{13}$; -C(=O)$OR^{13}$; -C(=O)$NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;

wherein $\mathbf{R^3}$ and $\mathbf{R^4}$ can form $=CH_2$ or $=CF_2$;
wherein $\mathbf{R^5}$ and $\mathbf{R^6}$ can form $=CH_2$ or $=CF_2$;
wherein $\mathbf{R^3}$ and $\mathbf{R^4}$ can form a $C_{3-7}$cycloalkyl, $C_{3-7}$cycloalkenyl, or a Het;
wherein $\mathbf{R^4}$ and $\mathbf{R^5}$ can form a $C_{3-7}$cycloalkyl, C3-7cycloalkenyl, or a Het;

wherein **$R^5$** and **$R^6$** can form a $C_{3-7}$cycloalkyl, C3-7cycloalkenyl, or a Het;
wherein **$R^6$** and **$R^7$** can form a $C_{3-7}$cycloalkyl, C3-7cycloalkenyl, or a Het;
wherein **$R^4$** and **$R^7$** can form a $C_{3-7}$cycloalkyl optionally substituted with oxo; $C_{3-7}$cycloalkenyl; or a Het;

**$R^8$** is methanediyl;
**$R^9$** is -O-C(=O)$NR^{11}R^{12}$;
**$R^{10}$** is hydrogen, $C_{1-8}$alkyl, -C(=O)$C_{1-8}$alkyl, -C(=O)-N($C_{1-8}$alkyl)$_2$, -C(=O)-N(cyclo$C_{3-7}$alkyl), or Het;
**$R^{11}$** and **$R^{12}$** each independently are hydrogen; $C_{1-6}$alkyl optionally substituted with one, two, or three substituents selected from oxo, -$OR^{13}$, $C_{3-7}$cycloalkyl, aryl, and Het; halo$C_{1-6}$alkyl; $C_{2-6}$alkenyl; $C_{2-6}$alkynyl; aryl; Het; $C_{3-7}$cycloalkyl optionally substituted with $C_{1-6}$alkyl; with the proviso that at least one $R^{11}$ or $R^{12}$ is not hydrogen; or **$R^{11}$** and **$R^{12}$** together with the nitrogen atom to which they are attached form a Het ring;
**$R^{13}$** is hydrogen; $C_{2-6}$alkenyl; $C_{2-6}$alkynyl; aryl; Het; mono- or di$C_{1-6}$alkylamino; $C_{3-7}$cycloalkyl optionally substituted with $C_{1-6}$alkyl; or $C_{1-6}$alkyl optionally substituted with one, two, or three substituents selected from hydroxy, cyano, $C_{1-4}$alkoxy, -O-P(=O)(-$OR^{14}$)(-$OR^{14}$), $C_{3-7}$cycloalkyl, aryl, and Het;
**$R^{14}$** is hydrogen, $C_{1-8}$alkyl, or halo$C_{1-8}$alkyl;
**$R^{15}$** is hydrogen, halo, $C_{1-6}$alkyl, $C_{2-6}$alkenyl, $C_{2-6}$alkynyl, amino, or oxo; and
**$R^{16}$** is hydrogen, halo, $C_{1-6}$alkyl, $C_{2-6}$alkenyl, $C_{2-6}$alkynyl, amino, or oxo;
each Het is independently a saturated, partially unsaturated or completely unsaturated monocycle, bicycle, or tricycle having 3 to 12 carbon atoms and containing 1, 2, 3, or 4 heteroatoms each independently selected from nitrogen, oxygen, and sulfur, said heterocycle being optionally substituted with one, two, three, or four substituents each independently selected from halo; oxo; nitro; cyano; $C_{1-6}$alkyl optionally substituted with hydroxy, $C_{1-6}$alkoxy, cyano, amino, aryl, -C(=O)-morpholinyl, or pyridinyl; $C_{2-6}$alkenyl; $C_{2-6}$alkynyl; -$OR^{13}$; -C(=O)$R^{13}$; -C(=O)O$R^{13}$; - S(=O)$_2R^{13}$; amino; mono- or di$C_{1-6}$alkylamino; azido; mercapto; halo$C_{1-6}$alkyl; halo$C_{1-6}$alkoxy; $C_{1-6}$alkylthio, $C_{3-7}$cycloalkyl; aryl; pyrazinyl; pyrrolidinyl; piperidinyl; pyrimidinyl; thieno[2,3-d]pyrimidinyl; piperazinyl; and morpholinyl; and
each **aryl** as a group or part of a group is an optionally fused phenyl, naphthyl, or anthracenyl; the optional fusing occurring with one or two $C_{3-7}$cycloalkyls; wherein said aryl as a group is optionally substituted with one, two, or three substituents selected from halo, hydroxy, nitro, cyano, carboxyl, $C_{1-6}$alkyl, $C_{2-6}$alkoxy, $C_{1-6}$alkoxy$C_{1-6}$alkyl, $C_{1-6}$alkylcarbonyl, amino, mono- or di$C_{1-6}$alkylamino, azido, mercapto,-S(=O)$_2R^{13}$, halo$C_{1-6}$alkyl, halo$C_{1-6}$alkoxy, cyclopropyl, pyrrolidinyl, piperidinyl, piperazinyl, 4-$C_{1-6}$alkylpiperazinyl, 4-$C_{1-6}$alkylcarbonylpiperazinyl, and morpholinyl; and wherein the morpholinyl and piperidinyl groups may optionally substituted with one or two $C_{1-6}$alkyls.

The present invention further relates to a pharmaceutical composition comprising a carrier, and as active ingredient an effective amount of a compound as defined in any one of the embodiments presented herein.
The present invention relates to a compound as defined in any one of the embodiments presented herein, for use as a medicament.
The present invention relates to a compound as defined in any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-0-trimethyladenosine; for use in the treatment of a disease selected from cancer, metabolic diseases, rheumatoid arthritis, psoriasis, inflammatory bowel disease, Vogt-Koyanagi-Harada syndrome (non-infectious blindness), and Alzheimer.
The present invention relates to a compound as defined in any one of the embodiments presented herein, for use in the treatment of a cardiovascular disease, with the proviso that the compound is not 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyiadenosine.
A method of inhibiting protein kinase activity in a warm-blooded animal comprises the administration to an animal in need thereof, of a kinase-inhibitory effective amount of a compound according to any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine.
A method of treating a disease selected from cancer, metabolic diseases, rheumatoid arthritis, psoriasis, inflammatory bowel disease, Vogt-Koyanagi-Harada syndrome (non-infectious blindness), and Alzheimer, in a warm-blooded animal comprises the administration to an animal in need thereof of an effective amount of a compound according to any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyiadenosine.
A method of treating a cardiovascular disease, in a warm-blooded animal comprises the administration to an animal in need thereof of an effective amount of a compound according to any one of the embodiments presented herein.

**Brief description of the drawings**

Figure 1 shows the nuclear magnetic resonance (NMR) spectrum of the compound C12.
Figure 2 shows the NMR spectrum of compound C95.
Figure 3 shows the NMR spectrum of compound C93.
Figure 4 shows the NMR spectrum of compound C78.
Figure 5 shows the NMR spectrum of compound C25.

**Detailed description of the invention**

The present invention relates to an *in vitro* method of inhibiting protein kinase activity which comprises contacting a protein kinase with a compound of formula (II), or the N-oxide, pharmaceutically acceptable salt, pharmaceutically acceptable solvate, or stereoisomer thereof,

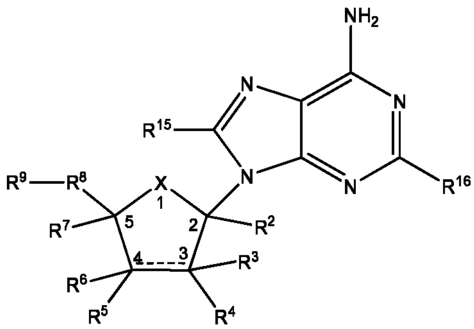

**(II)**

wherein

the dash bond (represented by ------ ) represents an optional double bond; provided that when there is a double bond in the 5-membered ring, the substituents $\mathbf{R^3}$ and $\mathbf{R^6}$ are not present;
X is selected from O and S;
$\mathbf{R^2}$ is selected from hydrogen; Het; aryl; $C_{1-6}$alkyl optionally substituted with halo, hydroxy, or Het; halo$C_{1-6}$alkyl; $C_{2-6}$alkenyl; $C_{2-6}$alkynyl; - C(=O)$NR^{11}R^{12}$; and a cyclo$C_{3-7}$alkyl or Het which is formed with any one of the carbon atoms of the depicted cyclopentane ring except carbon atom 2;
$\mathbf{R^3}$, $\mathbf{R^4}$, $\mathbf{R^5}$, $\mathbf{R^6}$, **and** $\mathbf{R^7}$ are each independently selected from hydrogen; halo; cyano; oxo; -$NR^{11}R^{12}$; -$OR^{13}$; -C(=O)$OR^{13}$; -C(=O)$NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;

wherein $\mathbf{R^3}$ and $\mathbf{R^4}$ can form =$CH_2$ or =$CF_2$;
wherein $\mathbf{R^5}$ and $\mathbf{R^6}$ can form =$CH_2$ or =$CF_2$;
wherein $\mathbf{R^3}$ and $\mathbf{R^4}$ can form a $C_{3-7}$cycloalkyl, C3-7cycloalkenyl, or a Het;
wherein $\mathbf{R^4}$ and $\mathbf{R^5}$ can form a $C_{3-7}$cycloalkyl, C3-7cycloalkenyl, or a Het;
wherein $\mathbf{R^5}$ and $\mathbf{R^6}$ can form a $C_{3-7}$cycloalkyl, C3-7cycloalkenyl, or a Het;
wherein $\mathbf{R^6}$ and $\mathbf{R^7}$ can form a $C_{3-7}$cycloalkyl, C3-7cycloalkenyl, or a Het;
wherein $\mathbf{R^4}$ and $\mathbf{R^7}$ can form a $C_{3-7}$cycloalkyl optionally substituted with oxo; $C_{3-7}$cycloalkenyl; or a Het;

$\mathbf{R^8}$ **is** methanediyl;
$\mathbf{R^9}$ is -O-C(=O)$NR^{11}R^{12}$;
$\mathbf{R^{10}}$ is hydrogen, $C_{1-8}$alkyl, -C(=O)$C_{1-8}$alkyl, -C(=O)-N($C_{1-8}$alkyl)$_2$, -C(=O)-N(cyclo$C_{3-7}$alkyl), or Het;
$\mathbf{R^{11}}$ and $\mathbf{R^{12}}$ each independently are hydrogen; $C_{1-6}$alkyl optionally substituted with one, two, or three substituents selected from oxo, -$OR^{13}$, $C_{3-7}$cycloalkyl, aryl, and Het; halo$C_{1-6}$alkyl; $C_{2-6}$alkenyl; $C_{2-6}$alkynyl; aryl; Het; $C_{3-7}$cycloalkyl optionally substituted with $C_{1-6}$alkyl; with the proviso that at least one $R^{11}$ or $R^{12}$ is not hydrogen; or
$\mathbf{R^{11}}$ and $\mathbf{R^{12}}$ together with the nitrogen atom to which they are attached form a Het ring;
$\mathbf{R^{13}}$ is hydrogen; $C_{2-6}$alkenyl; $C_{2-6}$alkynyl; aryl; Het; mono- or di$C_{1-6}$alkylamino; $C_{3-7}$cycloalkyl optionally substituted with $C_{1-6}$alkyl; or $C_{1-6}$alkyl optionally substituted with one, two, or three substituents selected from hydroxy, cyano, $C_{1-4}$alkoxy, -O-P(=O)(-$OR^{14}$)(-$OR^{14}$), $C_{3-7}$cycloalkyl, aryl, and Het;

**$R^{14}$** is hydrogen, $C_{1-6}$alkyl, or halo$C_{1-8}$alkyl;
**$R^{15}$** is hydrogen, halo, $C_{1-6}$alkyl, $C_{2-6}$alkenyl, $C_{2-6}$alkynyl, amino, or oxo; and
**$R^{16}$** is hydrogen, halo, $C_{1-6}$alkyl, $C_{2-6}$alkenyl, $C_{2-6}$alkynyl, amino, or oxo;
each **Het** is independently a saturated, partially unsaturated or completely unsaturated monocycle, bicycle, or tricycle having 3 to 12 carbon atoms and containing 1, 2, 3, or 4 heteroatoms each independently selected from nitrogen, oxygen, and sulfur, said heterocycle being optionally substituted with one, two, three, or four substituents each independently selected from halo; oxo; nitro; cyano; $C_{1-6}$alkyl optionally substituted with hydroxy, $C_{1-6}$alkoxy, cyano, amino, aryl, -C(=O)-morpholinyl, or pyridinyl; $C_{2-6}$alkenyl; $C_{2-6}$alkynyl; -$OR^{13}$; -C(=O)$R^{13}$; -C(=O)$OR^{13}$; - $S(=O)_2R^{13}$; amino; mono- or di$C_{1-6}$alkylamino; azido; mercapto; halo$C_{1-6}$alkyl; halo$C_{1-6}$alkoxy; $C_{1-6}$alkylthio, $C_{3-7}$cycloalkyl; aryl; pyrazinyl; pyrrolidinyl; piperidinyl; pyrimidinyl; thieno[2,3-d]pyrimidinyl; piperazinyl; and morpholinyl; and
each **aryl** as a group or part of a group is an optionally fused phenyl, naphthyl, or anthracenyl; the optional fusing occurring with one or two $C_{3-7}$cycloalkyls; wherein said aryl as a group is optionally substituted with one, two, or three substituents selected from halo, hydroxy, nitro, cyano, carboxyl, $C_{1-6}$alkyl, $C_{2-6}$alkoxy, $C_{1-6}$alkoxy$C_{1-6}$alkyl, $C_{1-6}$alkylcarbonyl, amino, mono- or di$C_{1-6}$alkylamino, azido, mercapto,-$S(=O)_2R^{13}$, halo$C_{1-6}$alkyl, halo$C_{1-6}$alkoxy, cyclopropyl, pyrrolidinyl, piperidinyl, piperazinyl, 4-$C_{1-6}$alkylpiperazinyl, 4-$C_{1-6}$alkylcarbonylpiperazinyl, and morpholinyl; and wherein the morpholinyl and piperidinyl groups may optionally substituted with one or two $C_{1-6}$alkyls.

In one embodiment, in the *in vitro* method of inhibiting protein kinase activity according to the present invention, the protein kinase is selected from the group consisting of ABL1, ACVR1B (ALK4), AKT1 (PKB alpha), AMPK A1/B1/G1, AURKA (Aurora A), BTK, CDK1/cyclin B, CHEK1 (CHK1), CSNK1G2 (CK1 gamma 2), CSNK2A1 (CK2 alpha 1), DYRK3, EGFR (ErbB1), EPHA2, ERBB2 (HER2), FGFR1, FLT3, FRAP1 (mTOR), GSK3B (GSK3 beta), IGF1R, IKBKB (IKK beta), INSR, IRAK4, JAK3, KDR (VEGFR2), KIT, LCK, MAP2K1 (MEK1), MAP4K4 (HGK), MAPK1 (ERK2), MAPK14 (p38 alpha), MAPK3 (ERK1), MAPK8 (JNK1), MARK2, MET (cMet), NEK1, PAK4, PDGFRB (PDGFR beta), PHKG2, PIM1, PLK1, PRKACA (PKA), PRKCB1 (PKC beta I), ROCK1, RPS6KA3 (RSK2), RPS6KB1 (p70S6K), SRC, SYK, and TEK (Tie2).

In one embodiment, in the *in vitro* method of inhibiting protein kinase activity according to the present invention,

**$R^2$ is hydrogen; or $C_{1-6}$alkyl optionally substituted with halo or hydroxy.**

In one embodiment, in the *in vitro* method of inhibiting protein kinase activity according to the present invention, compound of formula (II) is a compound of formula (III)

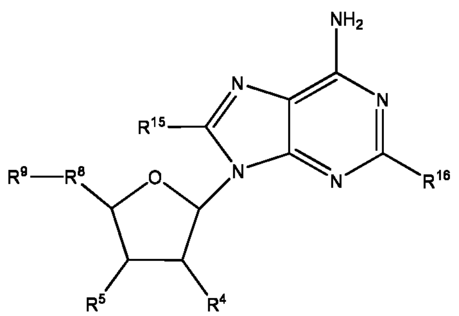

(III)

wherein **$R^4$**, **$R^5$**, **$R^8$**, **$R^9$**, **$R^{15}$**, and **$R^{16}$** are as defined above.

In one embodiment, in the *in vitro* method of inhibiting protein kinase activity according to the present invention, compound of formula (II) is a compound of formula (IV)

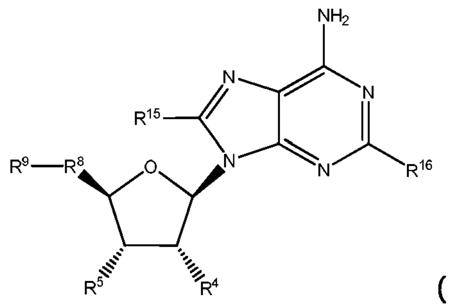

(IV)

wherein **$R^4$**, **$R^5$**, **$R^8$**, **$R^9$**, **$R^{15}$**, and **$R^{16}$** are as defined above.

In one embodiment, in the *in vitro* method of inhibiting protein kinase activity according to the present invention, compound of formula (II) is a compound of formula (V)

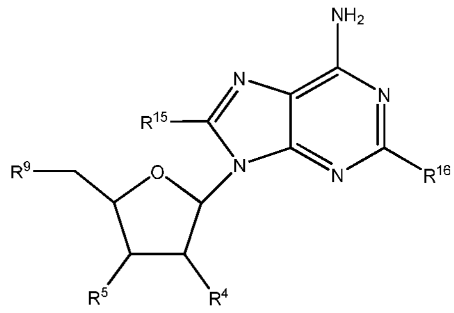

(V)

wherein **$R^4$**, **$R^5$**, **$R^9$**, **$R^{15}$**, and **$R^{16}$** are as defined above.

In one embodiment, in the *in vitro* method of inhibiting protein kinase activity according to the present invention, compound of formula (II) is a compound of formula (VI)

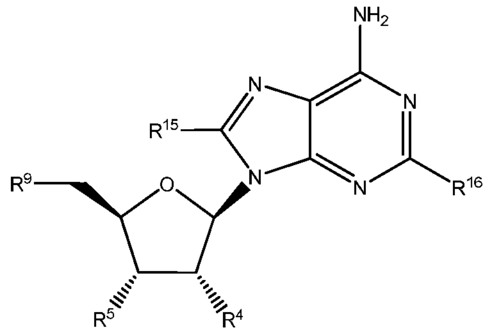

(VI)

wherein **$R^4$**, **$R^5$**, **$R^9$**, **$R^{15}$**, and **$R^{16}$** are as defined above.

The present invention further relates to a compound of formula (II), or the N-oxide, pharmaceutically acceptable salt, pharmaceutically acceptable solvate, or stereoisomer thereof,

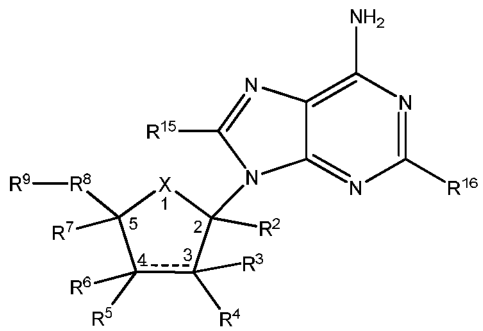

(II)

wherein

the dash bond (represented by ------ ) represents an optional double bond; provided that when there is a double bond in the 5-membered ring, the substituents **$R^3$** and **$R^6$** are not present;
**X** is selected from O and S;
**$R^2$** is selected from hydrogen; Het; aryl; $C_{1-6}$alkyl optionally substituted with halo, hydroxy, or Het; halo$C_{1-6}$alkyl; $C_{2-6}$alkenyl; $C_{2-6}$alkynyl; - C(=O)$NR^{11}R^{12}$; and a cyclo$C_{3-7}$alkyl or Het which is formed with any one of the carbon atoms of the depicted cyclopentane ring except carbon atom 2;
**$R^3$**, **$R^4$**, **$R^5$**, **$R^6$**, and **$R^7$** are each independently selected from hydrogen; halo; cyano; oxo; -$NR^{11}R^{12}$; -$OR^{13}$; -C(=O)$OR^{13}$; -C(=O)$NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;

wherein **$R^3$** and **$R^4$** can form =$CH_2$ or =$CF_2$;
wherein **$R^5$** and **$R^6$** can form =$CH_2$ or =$CF_2$;
wherein **$R^3$** and **$R^4$** can form a $C_{3-7}$cycloalkyl, $C_{3-7}$cycloalkenyl, or a Het;
wherein **$R^4$** and **$R^5$** can form a $C_{3-7}$cycloalkyl, $C_{3-7}$cycloalkenyl, or a Het;
wherein **$R^5$** and **$R^6$** can form a $C_{3-7}$cycloalkyl, $C_{3-7}$cycloalkenyl, or a Het;
wherein **$R^6$** and **$R^7$** can form a $C_{3-7}$cycloalkyl, $C_{3-7}$cycloalkenyl, or a Het;
wherein **$R^4$** and **$R^7$** can form a $C_{3-7}$cycloalkyl optionally substituted with oxo; $C_{3-7}$cycloalkenyl; or a Het;

**$R^8$** is methanediyl;
**$R^9$** is -O-C(=O)$NR^{11}R^{12}$; and
**$R^{10}$** is hydrogen, $C_{1-8}$alkyl, -C(=O)$C_{1-8}$alkyl, -C(=O)-N($C_{1-8}$alkyl)$_2$, -C(=O)-N(cyclo$C_{3-7}$alkyl), or Het;
**$R^{11}$** and **$R^{12}$** each independently are hydrogen; $C_{1-6}$alkyl optionally substituted with one, two, or three substituents selected from oxo, -$OR^{13}$, $C_{3-7}$cycloalkyl, aryl, and Het; halo$C_{1-6}$alkyl; $C_{2-6}$alkenyl; $C_{2-6}$alkynyl; aryl; Het; $C_{3-7}$cycloalkyl optionally substituted with $C_{1-6}$alkyl; with the proviso that at least one $R^{11}$ or $R^{12}$ is not hydrogen; or **$R^{11}$** and **$R^{12}$** together with the nitrogen atom to which they are attached form a Het ring;
**$R^{13}$** is hydrogen; $C_{2-6}$alkenyl; $C_{2-6}$alkynyl; aryl; Het; mono- or di$C_{1-6}$alkylamino; $C_{3-7}$cycloalkyl optionally substituted with $C_{1-6}$alkyl; or $C_{1-6}$alkyl optionally substituted with one, two, or three substituents selected from hydroxy, cyano, $C_{1-4}$alkoxy, -O-P(=O)(-$OR^{14}$)(-$OR^{14}$), $C_{3-7}$cycloalkyl, aryl, and Het;
**$R^{14}$** is hydrogen, $C_{1-6}$alkyl, or halo$C_{1-8}$alkyl;
**$R^{15}$** is hydrogen, halo, $C_{1-6}$alkyl, $C_{2-6}$alkenyl, $C_{2-6}$alkynyl, amino, or oxo; and
**$R^{16}$** is hydrogen, halo, $C_{1-6}$alkyl, $C_{2-6}$alkenyl, $C_{2-6}$alkynyl, amino, or oxo;
each **Het** is independently a saturated, partially unsaturated or completely unsaturated monocycle, bicycle, or tricycle having 3 to 12 carbon atoms and containing 1, 2, 3, or 4 heteroatoms each independently selected from nitrogen, oxygen, and sulfur, said heterocycle being optionally substituted with one, two, three, or four substituents each independently selected from halo; oxo; nitro; cyano; $C_{1-6}$alkyl optionally substituted with hydroxy, $C_{1-6}$alkoxy, cyano, amino, aryl, -C(=O)-morpholinyl, or pyridinyl; $C_{2-6}$alkenyl; $C_{2-6}$alkynyl; -$OR^{13}$; -C(=O)$R^{13}$; -C(=O)$OR^{13}$; - S(=O)$_2R^{13}$; amino; mono- or di$C_{1-6}$alkylamino; azido; mercapto; halo$C_{1-6}$alkyl; halo$C_{1-6}$alkoxy; $C_{1-6}$alkylthio, $C_{3-7}$cycloalkyl; aryl; pyrazinyl; pyrrolidinyl; piperidinyl; pyrimidinyl; thieno[2,3-d]pyrimidinyl; piperazinyl; and morpholinyl; and
each **aryl** as a group or part of a group is an optionally fused phenyl, naphthyl, or anthracenyl; the optional fusing occurring with one or two $C_{3-7}$cycloalkyls; wherein said aryl as a group is optionally substituted with one, two, or three substituents selected from halo, hydroxy, nitro, cyano, carboxyl, $C_{1-6}$alkyl, $C_{2-6}$alkoxy, $C_{1-6}$alkoxy$C_{1-6}$alkyl, $C_{1-6}$alkylcarbonyl, amino, mono- or di$C_{1-6}$alkylamino, azido, mercapto, -S(=O)$_2R^{13}$, halo$C_{1-6}$alkyl, halo$C_{1-6}$alkoxy, cyclopropyl, pyrrolidinyl, piperidinyl, piperazinyl, 4-$C_{1-6}$alkylpiperazinyl, 4-$C_{1-6}$alkylcarbonylpiperazinyl, and morpholinyl; and wherein the morpholinyl and piperidinyl groups may optionally substituted with one or two $C_{1-6}$alkyls;

with the proviso that the compound is not
5'-O-methyladenosine,
5'-O-n-butyladenosine,
2',5'-O-dimethyladenosine,
3',5'-O-dimethyladenosine,
2,5'-O-dimethyladenosine,
2-methyl-5'-O-n-butyladenosine,
$N^6$-5'-O-dimethyladenosine, or
2,$N^6$,5'-O-trimethyladenosine.

In one embodiment relating to a compound of formula (II), or the N-oxide, pharmaceutically acceptable salt,

pharmaceutically acceptable solvate, or stereoisomer thereof,
**$R^2$** is hydrogen; or $C_{1-6}$alkyl optionally substituted with halo or hydroxy.

In one embodiment, the present invention relates to a compound of formula (III), or the N-oxide, pharmaceutically acceptable salt, pharmaceutically acceptable solvate, or stereoisomer thereof,

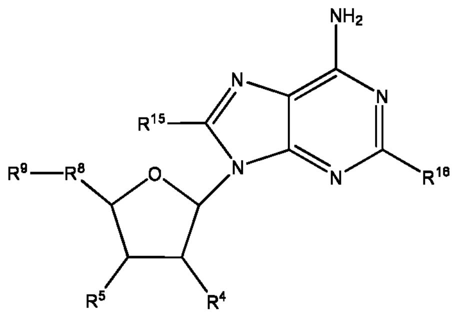

(III)

**$R^4$** and **$R^5$**, are each independently selected from hydrogen; halo; cyano; oxo; - $NR^{11}R^{12}$; $-OR^{13}$; $-C(=O)OR^{13}$; $-C(=O)NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;
wherein **$R^4$** and **$R^5$** can form a $C_{3-7}$cycloalkyl, C3-7cycloalkenyl, or a Het;

- **$R^8$** is methanediyl;
- **$R^9$** is-O-C(=O)$NR^{11}R^{12}$; and
- **$R^{15}$** is hydrogen, halo, $C_{1-6}$alkyl, $C_{2-6}$alkenyl, $C_{2-6}$alkynyl, amino, or oxo; and
- **$R^{16}$** is hydrogen, halo, $C_{1-6}$alkyl, $C_{2-6}$alkenyl, $C_{2-6}$alkynyl, amino, or oxo;

with the proviso that the compound is not

- 5'-O-methyladenosine,
- 5'-O-n-butyladenosine,
- 2',5'-O-dimethyladenosine,
- 3',5'-O-dimethyladenosine,
- 2,5'-O-dimethyladenosine,
- 2-methyl-5'-O-n-butyladenosine,
- $N^6$-5'-O-dimethyladenosine, or
- 2,$N^6$,5'-O-trimethyladenosine.

In one embodiment, the present invention relates to a compound of formula (IV), the N-oxide, pharmaceutically acceptable salt, pharmaceutically acceptable solvate, or stereoisomer thereof,

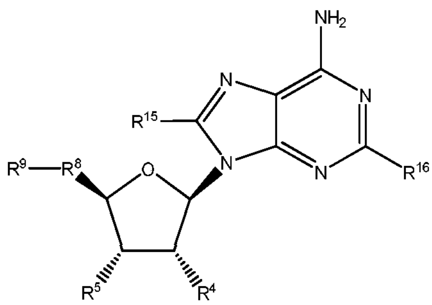

(IV)

wherein

**$R^4$** and **$R^5$** are each independently selected from hydrogen; halo; cyano; oxo; - $NR^{11}R^{12}$; $-OR^{13}$; $-C(=O)OR^{13}$; $-C(=O)NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;
wherein **$R^4$** and **$R^5$** can form a $C_{3-7}$cycloalkyl, C3-7cycloalkenyl, or a Het;

**$R^8$** is methanediyl;
**$R^9$** is -O-C(=O)$NR^{11}R^{12}$;
**$R^{15}$** is hydrogen, halo, $C_{1-6}$alkyl, $C_{2-6}$alkenyl, $C_{2-6}$alkynyl, amino, or oxo; and
**$R^{16}$** is hydrogen, halo, $C_{1-6}$alkyl, $C_{2-6}$alkenyl, $C_{2-6}$alkynyl, amino, or oxo;

with the proviso that the compound is not
5'-O-methyladenosine,
5'-O-n-butyladenosine,
2',5'-O-dimethyladenosine,
3',5'-O-dimethyladenosine,
2,5'-O-dimethyladenosine,
2-methyl-5'-O-n-butyladenosine,
$N^6$-5'-O-dimethyladenosine, or
2,$N^6$,5'-O-trimethyladenosine.

In one embodiment, the present invention relates to a compound of formula (V) or the N-oxide, pharmaceutically acceptable salt, pharmaceutically acceptable solvate, or stereoisomer thereof,

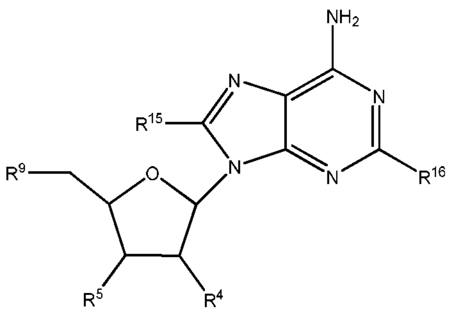

**(V)**

wherein

**$R^4$** and **$R^5$** are each independently selected from hydrogen; halo; cyano; oxo; - $NR^{11}R^{12}$; -$OR^{13}$; -C(=O)$OR^{13}$; -C(=O)$NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;
wherein **$R^4$** and **$R^5$** can form a $C_{3-7}$cycloalkyl, C3-7cycloalkenyl, or a Het;
**$R^9$** is -O-C(=O)$NR^{11}R^{12}$;
**$R^{15}$** is hydrogen, halo, $C_{1-6}$alkyl, $C_{2-6}$alkenyl, $C_{2-6}$alkynyl, amino, or oxo; and
**$R^{16}$** is hydrogen, halo, $C_{1-6}$alkyl, $C_{2-6}$alkenyl, $C_{2-6}$alkynyl, amino, or oxo;

with the proviso that the compound is not
5'-O-methyladenosine,
5'-O-n-butyladenosine,
2',5'-O-dimethyladenosine,
3',5'-O-dimethyladenosine,
2,5'-O-dimethyladenosine,
2-methyl-5'-O-n-butyladenosine,
$N^6$-5'-O-dimethyladenosine, or
2,$N^6$,5'-O-trimethyladenosine.

In one embodiment, the present invention relates to a compound of formula (VI), or the N-oxide, pharmaceutically acceptable salt, pharmaceutically acceptable solvate, or stereoisomer thereof,

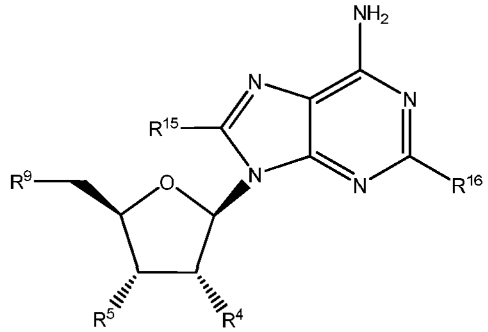

(VI)

wherein

**$R^4$** and **$R^5$** are each independently selected from hydrogen; halo; cyano; oxo; - $NR^{11}R^{12}$; $-OR^{13}$; $-C(=O)OR^{13}$; $-C(=O)NR^{11}R^{12}$; $C_{1-6}$alkyl optionally substituted with one or two hydroxy, halo, cyano, $C_{3-7}$cycloalkyl, aryl, or Het; halo$C_{1-6}$alkyl; $C_{2-6}$alkenyl; $C_{2-6}$alkynyl; and -SH;
wherein **$R^4$** and **$R^5$** can form a $C_{3-7}$cycloalkyl, C3-7cycloalkenyl, or a Het;
**$R^9$** is-$O-C(=O)NR^{11}R^{12}$;
**$R^{15}$** is hydrogen, halo, $C_{1-6}$alkyl, $C_{2-6}$alkenyl, $C_{2-6}$alkynyl, amino, or oxo; and
**$R^{16}$** is hydrogen, halo, $C_{1-6}$alkyl, $C_{2-6}$alkenyl, $C_{2-6}$alkynyl, amino, or oxo;

with the proviso that the compound is not
5'-O-methyladenosine,
5'-O-n-butyladenosine,
2',5'-O-dimethyladenosine,
3',5'-O-dimethyladenosine,
2,5'-O-dimethyladenosine,
2-methyl-5'-O-n-butyladenosine,
$N^6$-5'-O-dimethyladenosine, or
2,$N^6$,5'-O-trimethyladenosine.

The present invention further relates to a pharmaceutical composition comprising a carrier, and as active ingredient an effective amount of a compound as defined in any one of the embodiments presented herein.

The present invention relates to a compound as defined in any one of the embodiments presented herein, for use as a medicament.

The compound as defined in any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine, can be used in the treatment of a disease mediated by a protein kinase, wherein the protein kinase is selected from the group consisting of ABL1, ACVR1B (ALK4), AKT1 (PKB alpha), AMPK A1/B1/G1, AURKA (Aurora A), BTK, CDK1/cyclin B, CHEK1 (CHK1), CSNK1G2 (CK1 gamma 2), CSNK2A1 (CK2 alpha 1), DYRK3, EGFR (ErbB1), EPHA2, ERBB2 (HER2), FGFR1, FLT3, FRAP1 (mTOR), GSK3B (GSK3 beta), IGF1R, IKBKB (IKK beta), INSR, IRAK4, JAK3, KDR (VEGFR2), KIT, LCK, MAP2K1 (MEK1), MAP4K4 (HGK), MAPK1 (ERK2), MAPK14 (p38 alpha), MAPK3 (ERK1), MAPK8 (JNK1), MARK2, MET (cMet), NEK1, PAK4, PDGFRB (PDGFR beta), PHKG2, PIM1, PLK1, PRKACA (PKA), PRKCB1 (PKC beta I), ROCK1, RPS6KA3 (RSK2), RPS6KB1 (p70S6K), SRC, SYK, and TEK (Tie2).

The present invention relates to a compound as defined in any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine for use in the treatment of a disease selected from cancer, metabolic diseases, rheumatoid arthritis, psoriasis, inflammatory bowel disease, Vogt-Koyanagi-Harada syndrome (non-infectious blindness), and Alzheimer.

The present invention relates to a compound as defined in any one of the embodiments presented herein, for use in the treatment of a cardiovascular disease, with the proviso that the compound is not 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine.

A compound as defined in any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-0-trimethyladenosine, can be used for the manufacture of a medicament for the treatment of a disease selected from cancer, metabolic diseases, rheumatoid arthritis, psoriasis, inflammatory bowel disease, Vogt-Koyanagi-Harada syndrome (non-infectious blindness), and Alzheimer.

A compound as defined in any one of the embodiments presented herein, can be used for the manufacture of a medicament for the treatment of a cardiovascular disease.

A method of inhibiting protein kinase activity in a warm-blooded animal comprises the administration to an animal in need thereof, of a kinase-inhibitory effective amount of a compound according to any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine.

A method of inhibiting protein kinase activity in a warm-blooded animal comprises the administration to an animal in need thereof, of a kinase-inhibitory effective amount of a compound according to any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine, wherein the protein kinase is selected from the group consisting of ABL1, ACVR1B (ALK4), AKT1 (PKB alpha), AMPK A1/B1/G1, AURKA (Aurora A), BTK, CDK1/cyclin B, CHEK1 (CHK1), CSNK1G2 (CK1 gamma 2), CSNK2A1 (CK2 alpha 1), DYRK3, EGFR (ErbB1), EPHA2, ERBB2 (HER2), FGFR1, FLT3, FRAP1 (mTOR), GSK3B (GSK3 beta), IGF1R, IKBKB (IKK beta), INSR, IRAK4, JAK3, KDR (VEGFR2), KIT, LCK, MAP2K1 (MEK1), MAP4K4 (HGK), MAPK1 (ERK2), MAPK14 (p38 alpha), MAPK3 (ERK1), MAPK8 (JNK1), MARK2, MET (cMet), NEK1, PAK4, PDGFRB (PDGFR beta), PHKG2, PIM1, PLK1, PRKACA (PKA), PRKCB1 (PKC beta I), ROCK1, RPS6KA3 (RSK2), RPS6KB1 (p70S6K), SRC, SYK, and TEK (Tie2).

A method of treating a disease selected from cancer, metabolic diseases, rheumatoid arthritis, psoriasis, inflammatory bowel disease, Vogt-Koyanagi-Harada syndrome (non-infectious blindness), and Alzheimer, in a warm-blooded animal ca be used, said method comprises the administration to an animal in need thereof of an effective amount of a compound according to any one of the embodiments presented herein, including compounds 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-0-trimethyladenosine.

A method of treating a cardiovascular disease, in a warm-blooded animal can be used, said method comprises the administration to an animal in need thereof of an effective amount of a compound according to any one of the embodiments presented herein.

A method for preparing a compound of formula 5a, comprises: amination of a compound of formula 3a in the presence of a coupling agent and a suitable solvent;

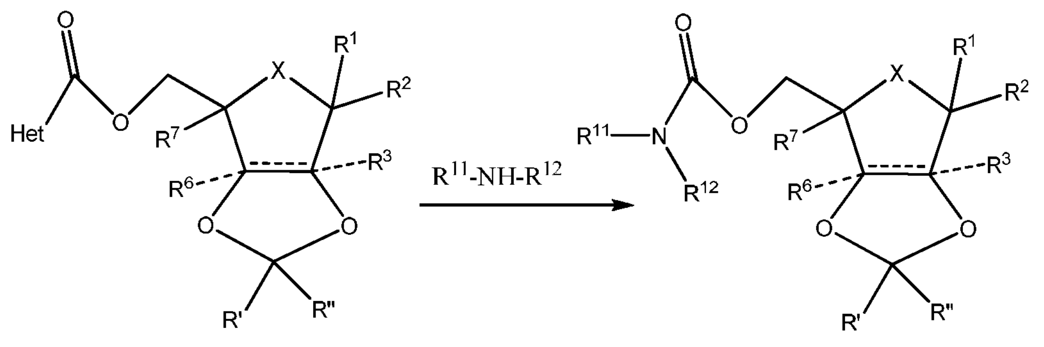


3a 4a

followed by deprotection of compound of formula 4a to obtain a compound of formula 5a,

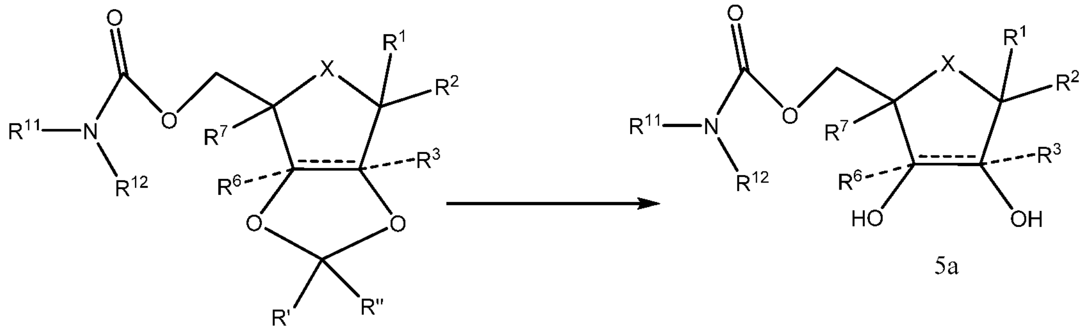

4a 5a

wherein

the dash bond, **X**, **$R^1$**, **$R^2$**, **$R^3$**, **$R^6$**, **$R^7$**, **$R^{11}$**, **$R^{12}$**, and **Het**, are as defined in claim 1;
**R'** is $C_{1-4}$alkyl or aryl; and
**R"** is hydrogen or $C_{1-4}$alkyl.

As used in the foregoing and hereinafter, the following definitions apply unless otherwise noted.

The term halo is generic to fluoro, chloro, bromo, and iodo.

The term "halo$C_{1-6}$alkyl" as a group or part of a group, e.g. in halo$C_{1-6}$alkoxy, is defined as mono- or halo substituted $C_{1-6}$alkyl, in particular $C_{1-6}$alkyl substituted with up to one, two, three, four, five, six, or more halo atoms, such as methyl or ethyl with one or more fluoro atoms, for example, difluoromethyl, trifluoromethyl, trifluoroethyl. Preferred is trifluoromethyl. Also included are perfluoro$C_{1-6}$alkyl groups, which are $C_{1-6}$alkyl groups wherein all hydrogen atoms are replaced by fluoro atoms, e.g. pentafluoroethyl. In case more than one halogen atom is attached to an alkyl group within the definition ofhalo$C_{1-6}$alkyl, the halogen atoms may be the same or different.

As used herein "$C_{1-4}$alkyl" as a group or part of a group defines straight or branched chain saturated hydrocarbon radicals having from 1 to 4 carbon atoms such as for example methyl, ethyl, 1-propyl, 2-propyl, I-butyl, 2-butyl, 2-methyl-1-propyl.

As used herein "$C_{1-6}$alkyl" encompasses $C_{1-4}$alkyl radicals and the higher homologues thereof having 5 or 6 carbon atoms such as, for example, 1-pentyl, 2-pentyl, 3-pentyl, 1-hexyl, 2-hexyl, 2-methyl-1-butyl, 2-methyl-1-pentyl, 2-ethyl-1-butyl, 3-methyl-2-pentyl, and the like. Of interest amongst $C_{1-6}$alkyl is $C_{1-4}$alkyl.

As used herein "$C_{1-8}$alkyl" encompasses $C_{1-6}$alkyl radicals and the higher homologues thereof having 7 or 8 carbon atoms such as, for example, heptyl, octyl, and branched versions like 3-ethyl-2-pentyl, 2-ethyl-1-hexyl.

The term "$C_{2-6}$alkenyl" as a group or part of a group defines straight and branched chained hydrocarbon radicals having saturated carbon-carbon bonds and at least one double bond, and having from 2 to 6 carbon atoms, such as, for example, ethenyl (or vinyl), 1-propenyl, 2-propenyl (or allyl), 1-butenyl, 2-butenyl, 3-butenyl, 2-methyl-2-propenyl, 2-pentenyl, 3-pentenyl, 2-hexenyl, 3-hexenyl, 4-hexenyl, 2-methyl-2-butenyl, 2-methyl-2-pentenyl and the like. Of interest amongst $C_{2-6}$alkenyl is $C_{2-4}$alkenyl.

As used herein "$C_{2-8}$alkenyl" encompasses $C_{2-6}$alkenyl radicals and the higher homologues thereof having 7 or 8 carbon atoms.

The term "$C_{2-6}$alkynyl" as a group or part of a group defines straight and branched chained hydrocarbon radicals having saturated carbon-carbon bonds and at least one triple bond, and having from 2 to 6 carbon atoms, such as, for example, ethynyl, 1-propynyl, 2-propynyl, 1-butynyl, 2-butynyl, 3-butynyl, 2-pentynyl, 3-pentynyl, 2-hexynyl, 3-hexynyl and the like. Of interest amongst $C_{2-6}$alkynyl is $C_{2-4}$alkynyl.

As used herein "$C_{2-8}$alkynyl" encompasses $C_{2-6}$alkynyl radicals and the higher homologues thereof having 7 or 8 carbon atoms.

$C_{3-7}$cycloalkyl is generic to cyclopropyl, cyclobutyl, cyclopentyl, cyclohexyl and cycloheptyl.

$C_{1-6}$alkoxy means $C_{1-6}$alkyloxy wherein $C_{1-6}$alkyl is as defined above.

$C_{1-8}$alkoxy means $C_{1-8}$alkyloxy wherein $C_{1-8}$alkyl is as defined above.

$C_{1-8}$alkanediyl defines bivalent straight and branched chain saturated hydrocarbon radicals having from 1 to 8 carbon atoms such as, for example, methylene, ethylene, 1,3-propanediyl, 1,4-butanediyl, 1,2-propanediyl, 2,3-butanediyl, 1,5-pentanediyl, 1,6-hexanediyl, 1,7-heptanediyl, 1,8-octanediyl, and the like. Of interest amongst $C_{1-8}$alkanediyls are $C_{1-6}$alkanediyl and $C_{1-4}$alkanediyl.

In a similar manner; $C_{2-8}$alkenediyl is defined as a bivalent straight and branched chain saturated hydrocarbon radical having from 2 to 8 carbon atoms and comprising at least one double bond between two of the carbon atoms. Of interest amongst $C_{2-8}$alkenediyls are $C_{2-6}$alkenediyl and $C_{2-4}$alkenediyl.

In a similar manner, $C_{2-8}$alkynediyl defines a bivalent straight and branched chain saturated hydrocarbon radical having from 1 to 8 carbon atoms with at least one triple bond between two carbon atoms. Of interest amongst $C_{2-8}$alkynediyls are $C_{2-6}$alkynediyl and $C_{2-4}$alkynediyl.

As used herein before, the term (=O) or oxo forms a carbonyl moiety when attached to a carbon atom, a sulfoxide moiety when attached to a sulfur atom and a sulfonyl moiety when two of said terms are attached to a sulfur atom. Whenever a ring or ring system is substituted with an oxo group, the carbon atom to which the oxo is linked is a saturated carbon.

"Amino", unless the context suggests otherwise, includes $NH_2$, $NHC_{1-6}$alkyl or $N(C_{1-6}alkyl)_2$, wherein in the amino definitions each $C_{1-6}$halkyl is especially $C_{1-3}$alkyl variants, or saturated cyclic amines such as pyrrolidinyl, piperidinyl, piperazinyl, 4-$C_{1-6}$alkylpiperazinyl, such as 4-methylpiperazinyl, 4-$C_{1-6}$alkylcarbonylpiperazinyl, and morpholinyl.

"Aryl" refers to any functional group or substituent derived from an aromatic ring, be it phenyl, naphthyl, thienyl, indolyl, and the like.

Typically, heterocycle moieties within the scope of the above definitions are thus a monocyclic ring with 5 or especially 6 ring atoms, or a bicyclic ring structure comprising a 6 membered ring fused to a 4, 5 or 6 membered ring. Typical such groups include $C_{3-8}$cycloalkyl, phenyl, benzyl, tetrahydronaphthyl, indenyl, indanyl, heterocyclyl such as from azepanyl, azocanyl, pyrrolidinyl, piperidinyl, morpholinyl, thiomorpholinyl, piperazinyl, indolinyl, pyranyl, tetrahydropyranyl, tetrahydrothiopyranyl, thiopyranyl, furanyl, tetrahydrofuranyl, thienyl, pyrrolyl, oxazolyl, isoxazolyl, thiazolyl, imidazolyl, pyridinyl, pyrimidinyl, pyrazinyl, pyridazinyl, tetrazolyl, pyrazolyl, indolyl, benzofuranyl, benzothienyl, benzimidazolyl, benzthiazolyl, benzoxazolyl, benzisoxazolyl, quinolinyl, tetrahydroquinolinyl, isoquinolinyl, tetrahydroisoquinolinyl, quinazolinyl, tetrahydroquinazolinyl and quinoxalinyl, any of which may be optionally substituted as defined herein.

The saturated heterocycle moiety thus includes radicals such as pyrrolinyl, pyrrolidinyl, pyrazolinyl, pyrazolidinyl, piperidinyl, morpholinyl, thiomorpholinyl, pyranyl, thiopyranyl, piperazinyl, indolinyl, azetidinyl, tetrahydropyranyl, tetrahydrothiopyranyl, tetrahydrofuranyl, hexahydropyrimidinyl, hexahydropyridazinyl, 1,4,5,6-tetrahydropyrimidinylamine, dihydro-oxazolyl, 1,2-thiazinanyl-1, I-dioxide, 1,2,6-thiadiazinanyl-1, I-dioxide, isothiazolidinyl-1, I-dioxide and imidazolidinyl-2,4-dione, whereas the unsaturated heterocycle include radicals with an aromatic character such as furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl. In each case the heterocycle may be condensed with a phenyl ring to form a bicyclic ring system.

The radical Het is a heterocycle as specified in this specification and claims. Examples of Het comprise, for example, pyrrolidinyl, piperidinyl, morpholinyl, thiomorpholinyl, piperazinyl, pyrrolyl, pyrazolyl, imidazolyl, oxazolyl, isoxazolyl, thiazinolyl, isothiazinolyl, thiazolyl, isothiazolyl, oxadiazolyl, thiadiazolyl, triazolyl (including 1,2,3-triazolyl, 1,2,4-triazolyl), tetrazolyl, furanyl, thienyl, pyridyl, pyrimidyl, pyridazinyl, pyrazolyl, triazinyl, and the like. Of interest amongst the Het radicals are those which are non-saturated, in particular those having an aromatic character. Of further interest are those Het radicals having one or two nitrogens.

Each of the Het radicals mentioned above may be optionally substituted with the number and kind of substituents mentioned in the definitions of the compounds of any of the subgroups of compounds of formula (II), (III), (IV), (V), and (VI). Some of the Het radicals mentioned in this and the following paragraph may be substituted with one, two or three hydroxy substituents. Such hydroxy substituted rings may occur as their tautomeric forms bearing keto groups. For example a 3-hydroxypyridazine moiety can occur in its tautomeric form 2H-pyridazin-3-one.

It should be noted that the radical positions on any molecular moiety used in the definitions may be anywhere on such moiety as long as it is chemically stable.

Radicals used in the definitions of the variables include all possible isomers unless otherwise indicated. For instance pyridyl includes 2-pyridyl, 3-pyridyl and 4-pyridyl ; pentyl includes 1-pentyl, 2-pentyl and 3-pentyl.

When any variable occurs more than one time in any constituent, each definition is independent. One embodiment comprises the compounds of any subgroup of compounds of formula (II)-(VI) specified herein, as well as the N-oxides, salts, as the possible stereoisomeric forms thereof. Another embodiment comprises the compounds of any subgroup of compounds of formula (II)-(VI) specified herein, as well as the salts as the possible stereoisomeric forms thereof.

The compounds of formula (II) have several centers of chirality and exist as stereochemically isomeric forms. The term "stereochemically isomeric forms" as used herein defines all the possible compounds made up of the same atoms bonded by the same sequence of bonds but having different three-dimensional structures which are not interchangeable, which the compounds of formula (II) may possess.

With reference to the instances where (R) or (S) is used to designate the absolute configuration of a chiral atom within a substituent, the designation is done taking into consideration the whole compound and not the substituent in isolation.

Unless otherwise mentioned or indicated, the chemical designation of a compound encompasses the mixture of all possible stereochemically isomeric forms, which said compound may possess. Said mixture may contain all diastereomers and/or enantiomers of the basic molecular structure of said compound. All stereochemically isomeric forms

of the compounds of the present invention both in pure form or mixed with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

Pure stereoisomeric forms of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphorsulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, if a specific stereoisomer is desired, said compound will be synthesized by stereospecific methods of preparation. These methods will advantageously employ enantiomerically pure starting materials.

The diastereomeric racemates of the compounds of formula (II) can be obtained separately by conventional methods. Appropriate physical separation methods that may advantageously be employed are, for example, selective crystallization and chromatography, e.g. column chromatography.

For some of the compounds of formula (II), N-oxides, salts, solvates, and the intermediates used in the preparation thereof, the absolute stereochemical configuration was not experimentally determined.

A person skilled in the art is able to determine the absolute configuration of such compounds using art-known methods such as, for example, X-ray diffraction.

The present invention is also intended to include all isotopes of atoms occurring on the present compounds. Isotopes include those atoms having the same atomic number but different mass numbers. By way of general example and without limitation, isotopes of hydrogen include tritium and deuterium. Isotopes of carbon include C-13 and C-14.

For therapeutic use, salts of the compounds of formula (II) are those wherein the counter-ion is pharmaceutically acceptable, which salts can be referred to as pharmaceutically acceptable acid and base addition salts. However, salts of acids and bases that are non-pharmaceutically acceptable may also find use, for example, in the preparation or purification of a pharmaceutically acceptable compound. All salts, whether pharmaceutically acceptable or not, are included within the ambit of the present invention.

The pharmaceutically acceptable acid and base addition salts as mentioned hereinabove are meant to comprise the therapeutically active nontoxic acid and base addition salt forms that the compounds of formula (II) are able to form. The pharmaceutically acceptable acid addition salts can conveniently be obtained by treating the base form with such appropriate acid in an anion form. Appropriate anions comprise, for example, trifluoroacetate, acetate, benzenesulfonate , benzoate, bicarbonate, bitartrate, bromide, calcium edetate, camsyiate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, nitrate, pamoate (embonate), pantothenate, phosphate/diphosphate, polygalacturonate, salicylate, stearate, subacetate, succinate, sulfate, tannate, tartrate, teoclate, triethiodide, and the like. The counterion of choice can be introduced using ion exchange resins. Conversely said salt forms can be converted by treatment with an appropriate base into the free base form.

The compounds of formula (II) containing an acidic proton may also be converted into their nontoxic metal or amine addition salt forms by treatment with appropriate organic and inorganic bases in a cation form. Appropriate basic salts comprise those formed with organic cations such as benzathine, chloroprocaine, choline, diethanolamine, ethylenediamine, meglumine, procaine, and the like; and those formed with metallic cations such as aluminum, calcium, lithium, magnesium, potassium, sodium, zinc, and the like. Conversely said salt forms can be converted by treatment with an appropriate acid into the free form.

The term addition salt as used hereinabove also comprises the solvates which the compounds of formula (II) as well as the salts thereof, are able to form. Such solvates are for example hydrates, alcoholates and the like.

The N-oxide forms of the present compounds are meant to comprise the compounds of formula (II) wherein one or several nitrogen atoms are oxidized to the so-called N-oxide.

It will be appreciated that the compounds of formula (II) may have metal binding, chelating, complex forming properties and therefore may exist as metal complexes or metal chelates. Such metalated derivatives of the compounds

of formula (II) are intended to be included within the scope of the present invention.

Some of the compounds of formula (II) may also exist in their tautomeric form. Such forms although not explicitly indicated in the above formula are intended to be included within the scope of the present invention.

The central "pentane" ring of a compound of formula (I)

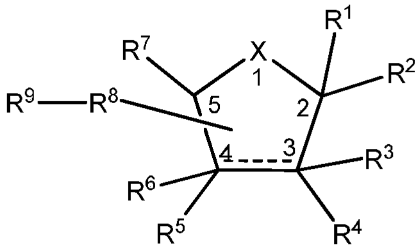

(I)

may be selected from:

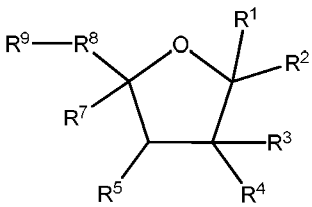
,
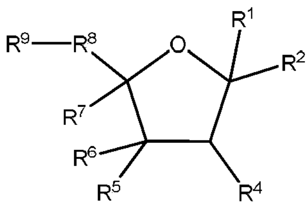
,
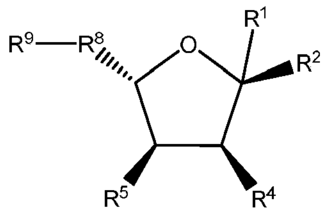
,

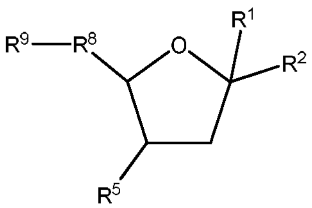
,
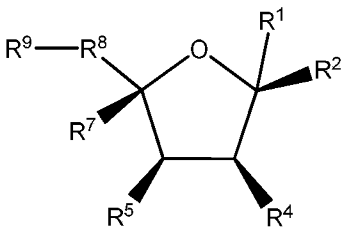
,
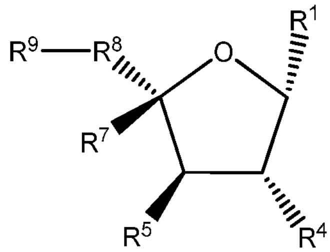
,

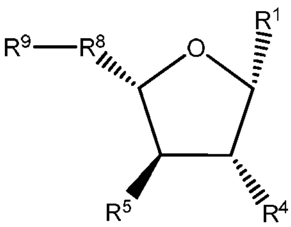
,
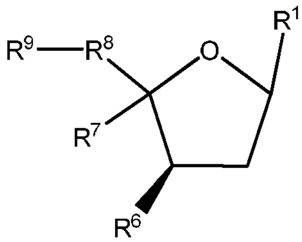
,
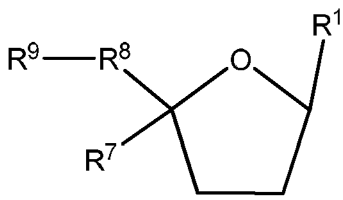
,

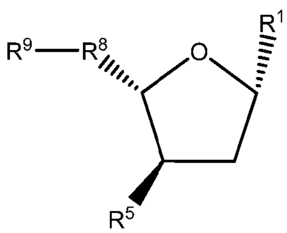
,
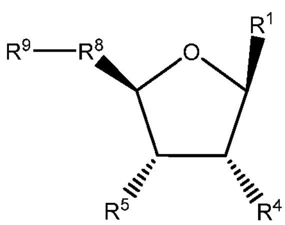
,
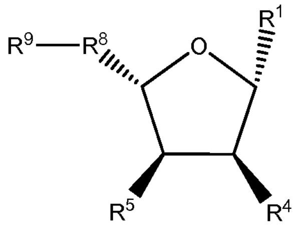
,

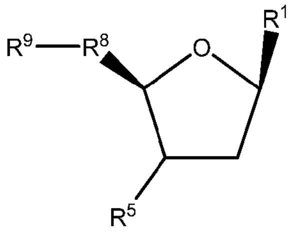,
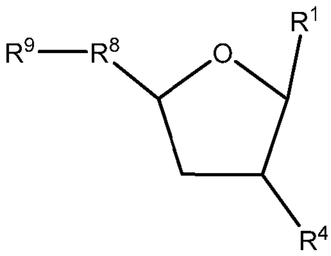,
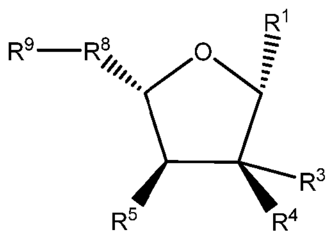,

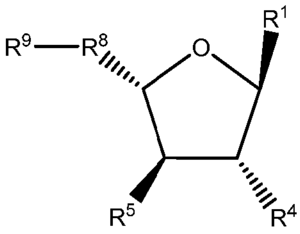,
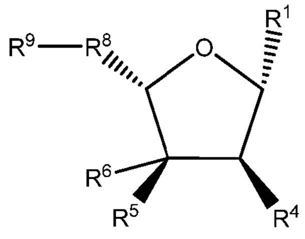,
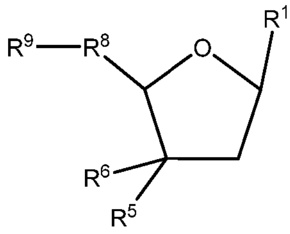,

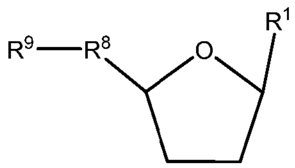,
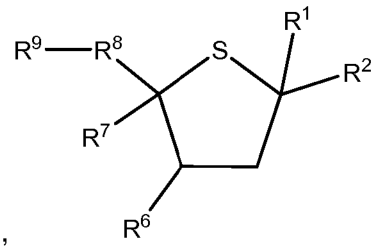,
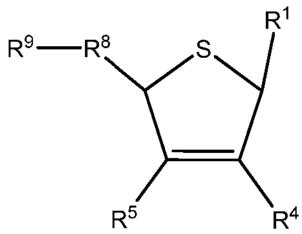,

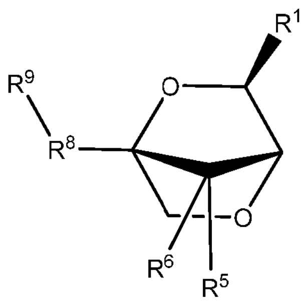,
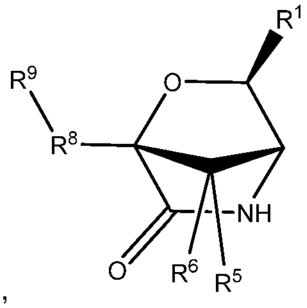,
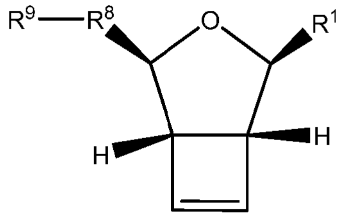,

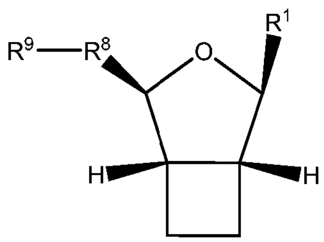,
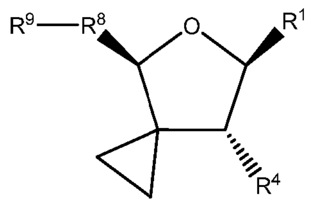,
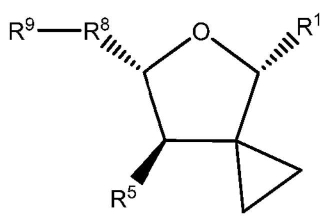,

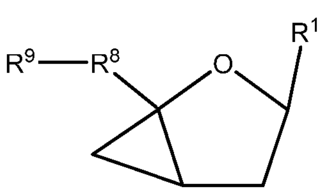,
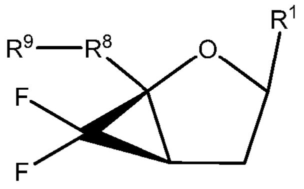,
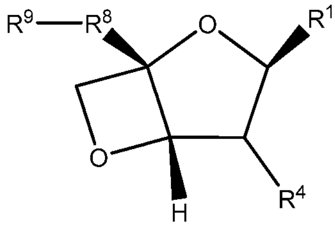,

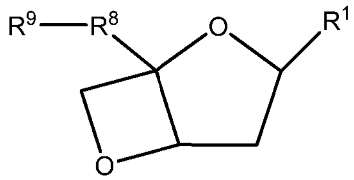 , 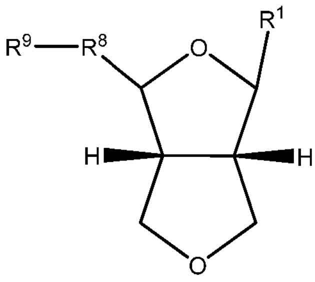 , 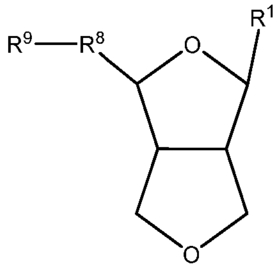 ,

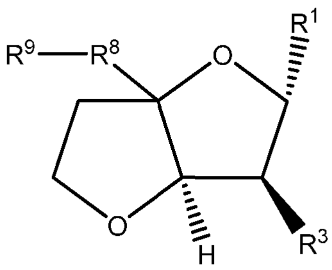 , and 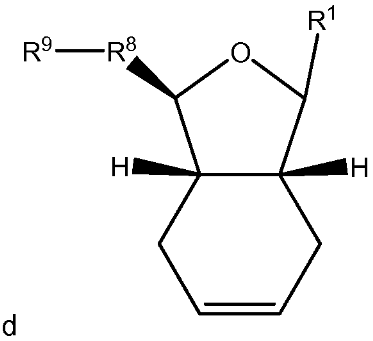 .

In one embodiment, $\mathbf{R^9}$ is -O-C(=O)NR$^{11}$R$^{12}$;

$\mathbf{R^{11}}$ is hydrogen; and

$\mathbf{R^{12}}$ is selected from 3-(1H-1,3-benzodiazol-2-yl)propan-1-amine; 2-(1-benzothiophen-3-yl)ethan-1-amine; (R)-4-chloro-a-methylbenzylamine; (1S,2R)-(-)-cis-1-amino-2-indanol; (2S)-1-methoxy-3-phenylpropan-2-amine; (S)-(-)-3-amino-1,2-propanediol; (3R)-1-benzylpyrrolidin-3-amine; 4-methylsulphonylbenzylamine; 1-(1,3-benzodioxol-5-yl)methanamine; 2-(aminomethyl)benzimidazole; 4-amino-1-isopropyl-piperidine; O-(2,3)dihydrobenzofuran-2-yl)methylamine; 1-(2-aminoethyl)2-imidazolidinone; 1-(2-aminoethyl)pyrrolidin-2-one; DL-1-amino-2-propanol; (aminomethyl)cyclopropane; 2-(4-methyl-piperazin-1-yl)ethylamine; 2-(2-aminoethyl)1-methylpyrrolidine; 2-(morpholin-4-yl)ethanamine; 2-(pyrrolidin-1-yl)ethanamine; 3-(1H-imidazol-1-yl)propan-1-amine; 3-amino-1-(4-methylpiperazin-1-yl)-propan-1-one; 3-(dimethylamino)-1-propylamine; dimethylethylenediamine; and N,N,N'-trimethylethylenediamine;

or $\mathbf{R^{11}}$ and $\mathbf{R^{12}}$ together with the nitrogen atom to which they are attached form a Het ring selected from 2S)-pyrrolidin-2-yl-methanol; (R)-3-hydroxypyrrolidine; (S)-(-)-3-(dimethylamino)-pyrrolidine; 4-N-benzyl-2-hydroxymethylpiperazine; 1-(2-chlorophenyl)piperazine; 1-(2-methoxyethyl)piperazine; 1-(2-phenylethyl)piperazine; 4-[2-(piperazin-1-yl)acetyl]-morpholine; 1-acetylpiperazine; 1-allylpiperazine; 1-(2-propyl)-piperazine; piperazin-1-yl-pyrrolidin-1-yl-methanone; 1-(tetrahydro-2-furylmethyl)piperazine; 1-acetyl-1,4-diazepane; 1-(2-hydroxyethyl)piperazine; 2-piperazinopyrimidine; 2-pyrrolidin-3-yl-pyrazine; 3-(4-fluorobenzyl)pyrrolidine; 3-piperazinopropionitrile; 3-pyrrolidin-2-ylmethyl-pyridine; 3,3-difluoroazetidine; 4-morpholinopiperidine; 4-methoxypiperidine; 2-hydroxymethylmorpholine; morpholin-4-yl-piperazin-1-yl-methanone; morpholine; piperazine-1-carbocyclic acid; 4-(dimethylamino)-piperidine; 1-(methylsulfonyl)spiro[indoline-3,4'-piperidine]; and thiomorpholine 1,1-dioxide.

Compounds of formula (I) can be prepared starting from compound 1a. If R$^3$ or R$^4$, and R$^5$ or R$^6$ comprise a -OH functional group to form an $\alpha$-diol, a step of protection is preferable. This may be done using a ketone, under acidic conditions. In the depicted ketone, R' may be selected preferably from $C_{1-4}$alkyl and aryl, and R" may be selected preferably from hydrogen and $C_{1-4}$alkyl. The intermediate 2a is formed after protection of compound 1a.

Reaction I: Protection

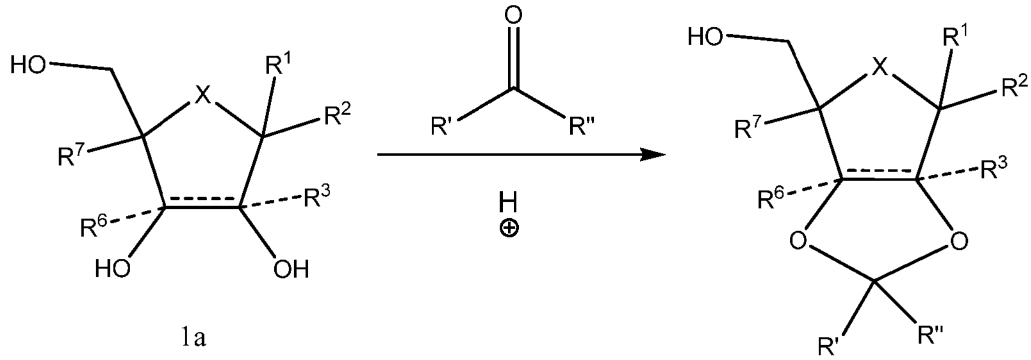


1a 2a

Intermediate 2a can be coupled with an amine by an amide forming reaction such as any of the procedures for the formation of an amide bond described hereinafter. In particular, 2a may be treated with a coupling agent, for example N, N'-carbonyldiimidazole (CDI), *N*-Ethoxycarbonyl-2-ethoxy-1,2-dihydroquinoline (EEDQ), *N*-isobutoxycarbonyl-2-isobutoxy-1,2-dihydroquinoline (IIDQ), 1-ethyl-3-(3-dimethylaminopropyl)-carbodiimide (EDCI), or benzotriazol-1-yl-oxy-tris-pyrrolidino-phosphonium hexafluorophosphate (commercially available as PyBOP®), in a solvent such as ether, e.g. THF, or a halogenated hydrocarbon, e.g. dicloromethane, chloroform, dichloroethane, to form the intermediate 3a, during reaction II.

Reaction II: Coupling

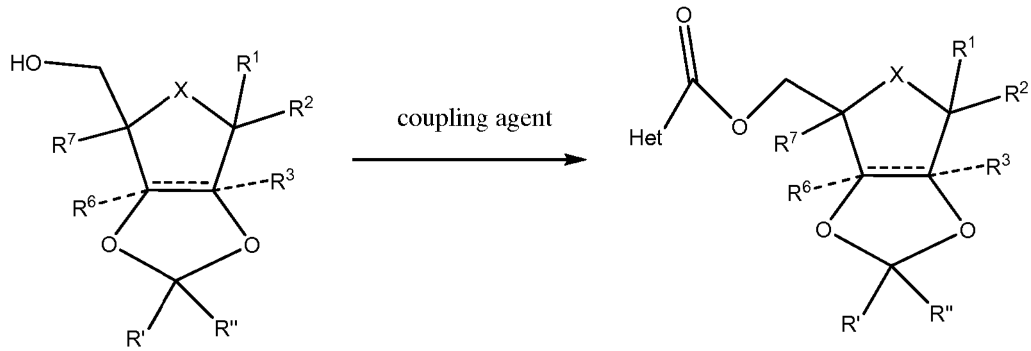


2a 3a

The intermediate 3a is then reacted with the desired amine (reaction III), preferably after reacting 2a with the coupling agent (reaction II). The reaction of 3a with the amine is preferably conducted in the presence of a base, for example a trialylamine such as triethylamine or diisopropylamine, or 1,8-diazabicyclo[5.4.0]undec-7-ene (DBU). This reaction leads to the formation of intermediate 4a.

Reaction III: Amination

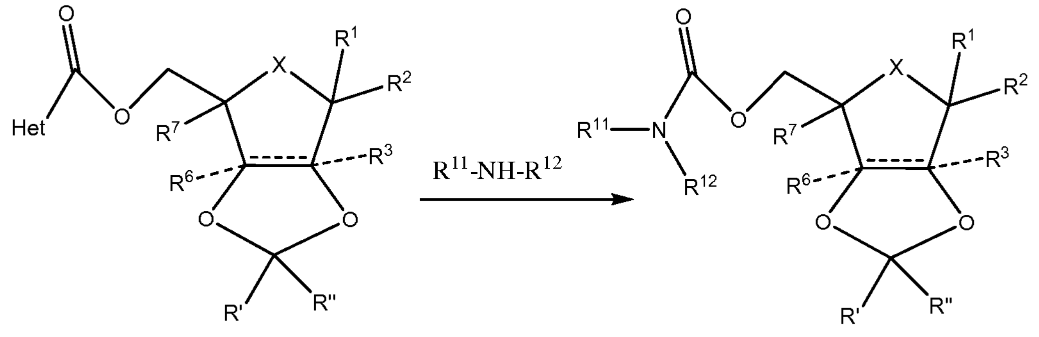


3a 4a

The intermediate 4a may then be deprotected. A suitable way of deprotecting the cyclic acetal group can be to perform an hydrolysis under acidic conditions. Therefore, an acid, e.g. trifluoroacteic acid (TFA) may be used in combination with $H_2O$. The resulting molecule is the carbamate 5a.

Reaction IV: Deprotection

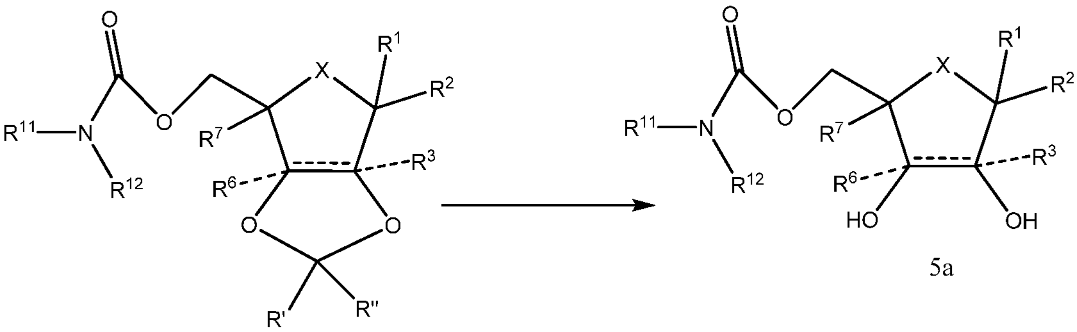

4a 5a

Compounds of formula (I) may be converted into each other following art-known functional group transformation reactions. For example, amino groups may be N-alkylated, nitro groups reduced to amino groups, a halo atom may be exchanged for another halo.

The compounds of formula (I) may be converted to the corresponding N-oxide forms following art-known procedures for converting a trivalent nitrogen into its N-oxide form. Said N-oxidation reaction may generally be carried out by reacting the starting material of formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert-butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Pure stereochemically isomeric forms of the compounds of formula (I) may be obtained by the application of art-known procedures. Diastereomers may be separated by physical methods such as selective crystallization and chromatographic techniques, e.g. counter-current distribution, liquid chromatography and the like.

The compounds of formula (I) may be obtained as racemic mixtures of enantiomers which can be separated from one another following art-known resolution procedures.

The racemic compounds of formula (I), which are sufficiently basic or acidic may be converted into the corresponding diastereomeric salt forms by reaction with a suitable chiral acid, respectively chiral base. Said diastereomeric salt forms are subsequently separated, for example, by selective or fractional crystallization and the enantiomers are liberated therefrom by alkali or acid. An alternative manner of separating the enantiomeric forms of the compounds of formula (I) involves liquid chromatography, in particular liquid chromatography using a chiral stationary phase. Said pure stereochemically isomeric forms may also be derived from the corresponding pure stereochemically isomeric forms of the appropriate starting materials, provided that the 15 reaction occurs stereospecifically. Preferably if a specific stereoisomer is desired, said compound may be synthesized by stereospecific methods of preparation. These methods may advantageously employ enantiomerically pure starting materials.

In a further aspect, the present invention concerns a pharmaceutical composition comprising a therapeutically effective amount of a compound of a compound of any of the subgroups of compounds of formula (II)-(VI) as specified herein, and a pharmaceutically acceptable carrier. A therapeutically effective amount in this context is an amount sufficient to prophylactically act against, to stabilize or reduce illnesses mediated by protein kinases in ill subjects or subjects being at risk of being ill, in particular a protein kinase selected from the group consisting of ABL1, ACVR1B (ALK4), AKT1 (PKB alpha), AMPK A1/B1/G1, AURKA (Aurora A), BTK, CDK1/cyclin B, CHEK1 (CHK1), CSNK1G2 (CK1 gamma 2), CSNK2A1 (CK2 alpha 1), DYRK3, EGFR (ErbB1), EPHA2, ERBB2 (HER2), FGFR1, FLT3, FRAP1 (mTOR), GSK3B (GSK3 beta), IGF1R, IKBKB (IKK beta), INSR, IRAK4, JAK3, KDR (VEGFR2), KIT, LCK, MAP2K1 (MEK1), MAP4K4 (HGK), MAPK1 (ERK2), MAPK14 (p38 alpha), MAPK3 (ERK1), MAPK8 (JNK1), MARK2, MET (cMet), NEK1, PAK4, PDGFRB (PDGFR beta), PHKG2, PIM1, PLK1, PRKACA (PKA), PRKCB1 (PKC beta I), ROCK1, RPS6KA3 (RSK2), RPS6KB1 (p70S6K), SRC, SYK, and TEK (Tie2). Examples of illnesses mediated by protein kinases include in particular cancer, metabolic diseases, rheumatoid arthritis, psoriasis, inflammatory bowel disease, cardiovascular diseases, Vogt-Koyanagi-Harada syndrome (non-infectious blindness), and Alzheimer. In still a further aspect, this invention relates to a process of preparing a pharmaceutical composition as specified herein, which comprises intimately mixing a pharmaceutically acceptable carrier with a therapeutically effective amount of a compound of any of the subgroups of compounds of formula (II)-(VI) as specified herein.

Therefore, the compounds of the present invention or any subgroup thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets.

Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations, which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

The compounds of the present invention may also be administered via oral inhalation or insufflation by means of methods and formulations employed in the art for administration via this way. Thus, in general the compounds of the present invention may be administered to the lungs in the form of a solution, a suspension or a dry powder, a solution being preferred. Any system developed for the delivery of solutions, suspensions or dry powders via oral inhalation or insufflation are suitable for the administration of the present compounds.

Thus, the present invention also provides a pharmaceutical composition adapted for administration by inhalation or insufflation through the mouth comprising a compound of formula (II) and a pharmaceutically acceptable carrier. Preferably, the compounds of the present invention are administered via inhalation of a solution in nebulized or aerosolized doses.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compounds of formula (II) show kinase inhibition properties. Illnesses and diseases treatable using the compounds and methods of the present invention include protein kinase mediated diseases like cancer, metabolic diseases, rheumatoid arthritis, psoriasis, inflammatory bowel disease, cardiovascular diseases, Vogt-Koyanagi-Harada syndrome (non-infectious blindness), and Alzheimer. Many of the compounds of this invention may show a favourable

pharmacokinetic profile and have attractive properties in terms of bioavailability, including an acceptable half-life, AUC (area under the curve) and peak values and lacking unfavourable phenomena such as insufficient quick onset and tissue retention.

The combinations of the present invention may be used as medicaments. Said use as a medicine or method of treatment comprises the systemic administration to ill subjects of an amount effective to combat the conditions associated with the illnesses. Consequently, the combinations of the present invention can be used in the manufacture of a medicament useful for treating, preventing or combating illness or disease associated with protein kinases including cancer, metabolic diseases, rheumatoid arthritis, psoriasis, inflammatory bowel disease, cardiovascular diseases, Vogt-Koyanagi-Harada syndrome (non-infectious blindness), and Alzheimer.

The term "therapeutically effective amount" as used herein means that amount of active compound or component or pharmaceutical agent that elicits the biological or medicinal response in a tissue, system, animal or human that is being sought, in the light of the present invention, by a researcher, veterinarian, medical doctor or other clinician, which includes alleviation of the symptoms of the disease being treated.

In general it is contemplated that an antiviral effective daily amount would be from 0.01 mg to 1500 mg daily, more preferably from 0.1 mg to 50 mg daily. It may be appropriate to administer the required dose as one, two, three, four or more (sub-)doses at appropriate intervals throughout the day. Said (sub-)doses may be formulated as unit dosage forms, for example, containing 1 to 1000 mg, and in particular 5 to 200 mg of active ingredient per unit dosage form.

The exact dosage and frequency of administration depends on the particular compound of formula (II) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines.

## Examples

### Example 1: Synthesis of compounds according to the present invention

The synthesis was performed according to general scheme below starting from 2',3'-O-isopropylideneadenosine purchased from Sigma-Aldrich, which was activated by carbonyldiimidazole in the presence of pyridine. The obtained imidazole intermediate was then reacted with the selected amines to form carbamates. Finally, the isopropylidene protecting group was removed by treatment with trifluoroacetic acid and the materials were purified by preparative high-performance liquid chromatography (HPLC) under basic conditions to give the final products as free amines. The synthetic scheme is outlined below. The structures of the final compounds and molecular weight can be found in the table 1 below.

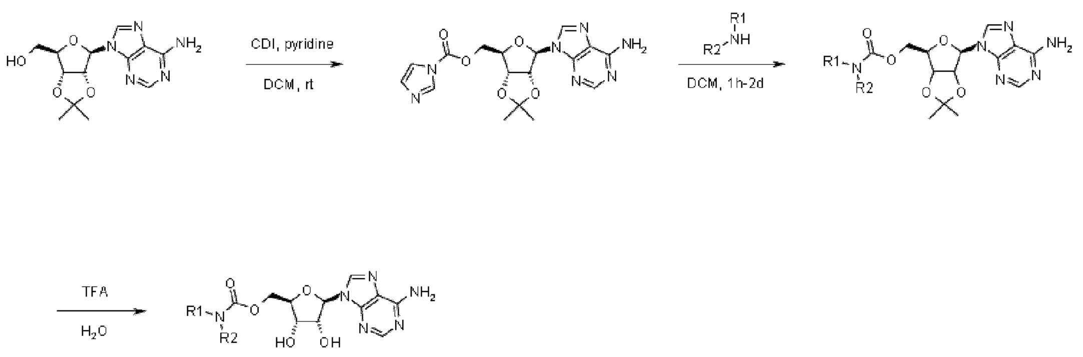


Carbonyldiimidazole (106 mg, 0.65 mmol, 2.0 eq) was dissolved in dichloromethane (2 mL) and pyridine (31 µL, 0.39 mmol, 1.2 eq) was added at room temperature. The mixture was stirred for 20 minutes and 2',3'-O-isopropylideneadenosine (100 mg, 0.32 mmol, 1.0 eq) was added. The mixture was then stirred for 1 hour at room temperature before the amine (0.36 mmol, 1.1 eq) was added. If the amine was added as HCl salt, an equimolar amount of triethylamine was added. The reaction mixture was stirred overnight at room temperature. All volatiles were removed in vacuo, 90% TFA in water (1 mL) was added at 0°C and the reaction was stirred for 2 hours at room temperature. The mixture was concentrated in vacuo and the residue was purified by preparative HPLC (gradient of $CH_3CN$ in 50 mM of $NH_4HCO_3$, at a pH value of 10) to give the final product as a free amine.

All final products were analyzed by high-performance liquid chromatography-mass spectrometry (HPLC-MS)

by two different liquid chromatography methods. NMR spectra were recorded for some of the compounds (cfr. figures 1-5).

Preparative HPLC was performed on a Gilson system equipped with a UV detector using an Xbridge Prep C-18 5 μm OBD, column. Analytical HPLC-MS was performed using an Agilent 1100 series Liquid Chromatograph/Mass Selective Detector equipped with an electrospray interface and a UV diode array detector. Analyses were performed by two methods using an ACE 3 C8 column with gradients of acetonitrile in 0.1% aqueous TFA and a flow of 1 mL/min, and using an Xbridge C18 column with gradients of acetonitrile in 10 mM of ammonium bicarbonate and a flow of 1 mL/min. 1H-NMR spectra was recorded on a Varian 400 MHz instrument at 25 °C.

Table 1

| Compound | Structure | Molecular Weight (g.mol-1) |
|---|---|---|
| C1 | 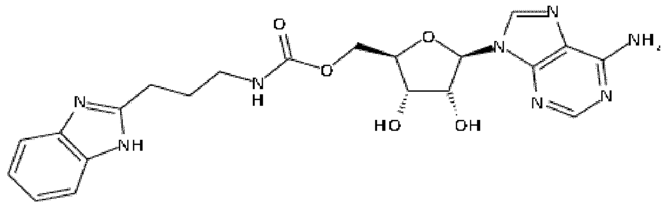 | 468.48 |
| C2 | 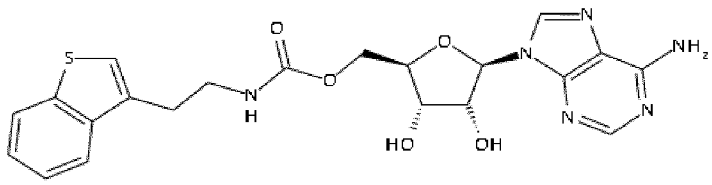 | 470.51 |
| C4 | 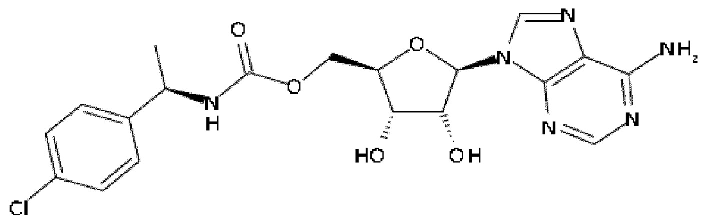 | 448.87 |
| C5 | 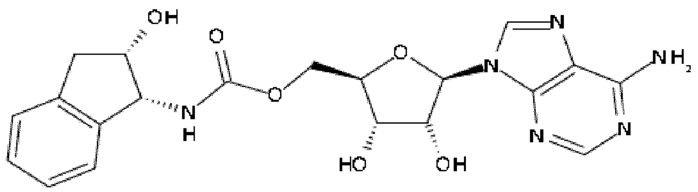 | 442.43 |
| C7 | 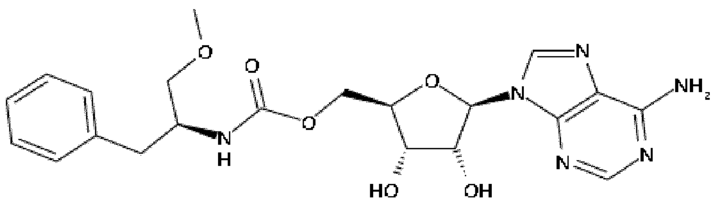 | 458.48 |
| C8 | 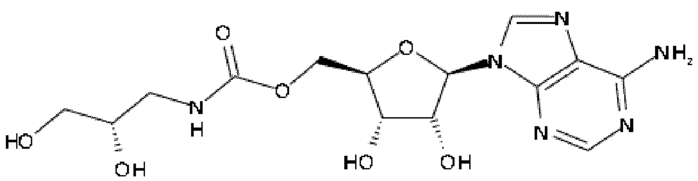 | 384.35 |

(continued)

| Compound | Structure | Molecular Weight (g.mol-1) |
|---|---|---|
| C9 | 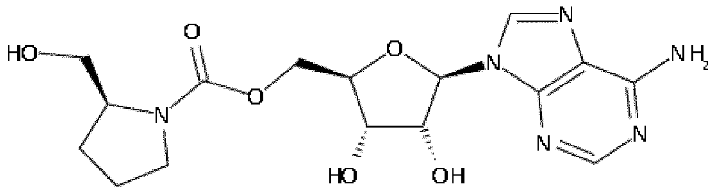 | 394.39 |
| C10 | 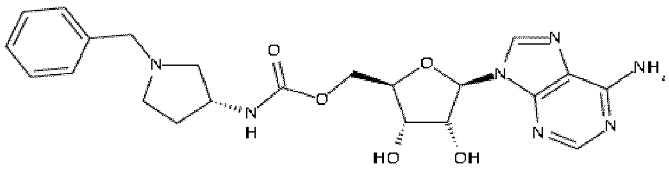 | 469.50 |
| C11 | 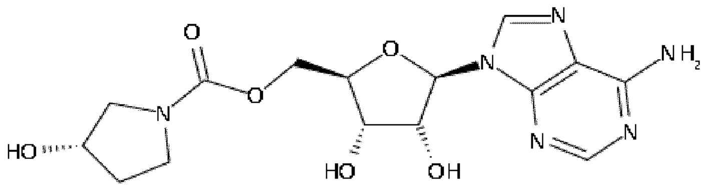 | 380.36 |
| C12 | 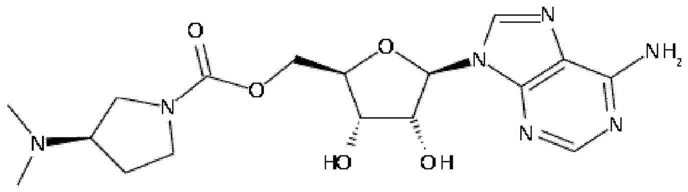 | 407.43 |
| C13 | 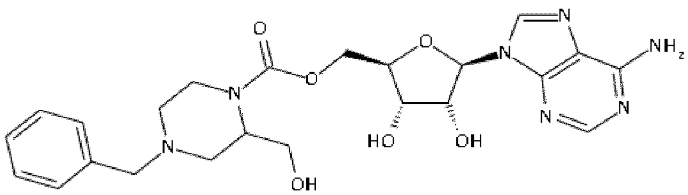 | 499.53 |
| C14 | 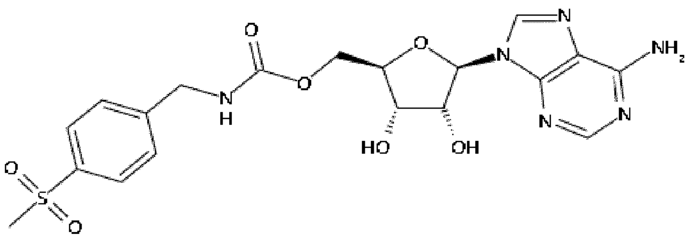 | 478.49 |
| C15 | 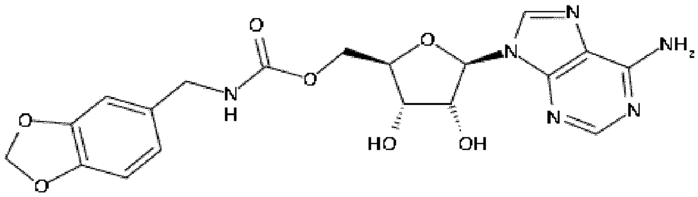 | 444.41 |

(continued)

| Compound | Structure | Molecular Weight (g.mol-1) |
|---|---|---|
| C17 | 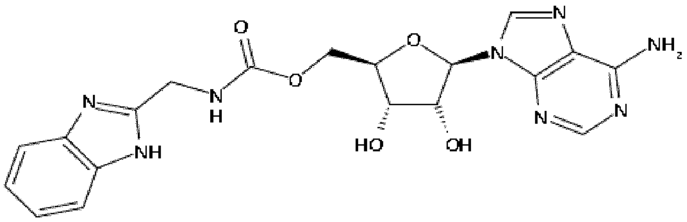 | 440.42 |
| C19 | 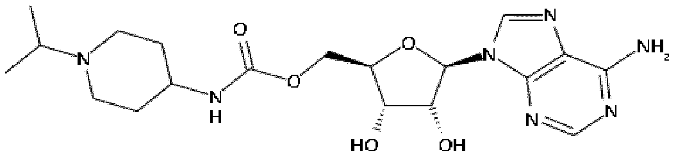 | 435.49 |
| C20 | 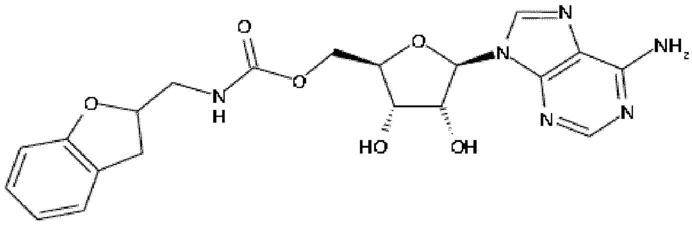 | 442.43 |
| C21 | 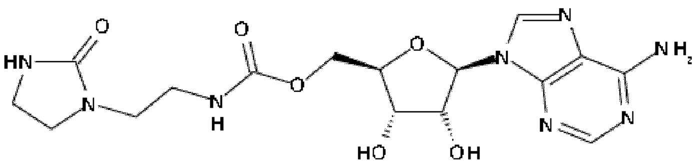 | 422.40 |
| C22 | 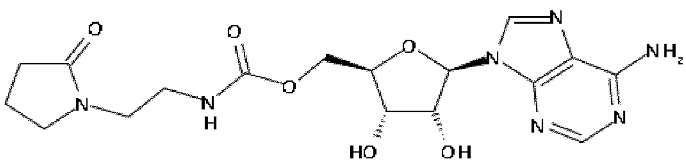 | 421.42 |
| C23 | 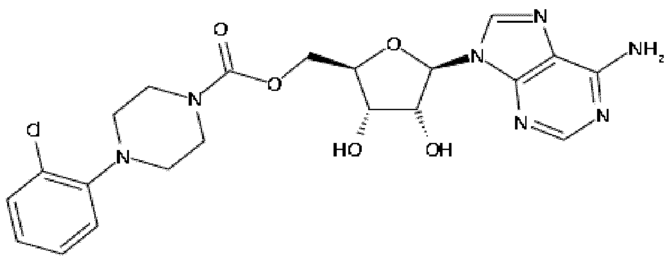 | 489.93 |
| C25 | 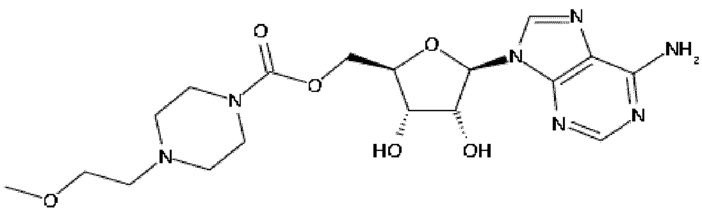 | 437.46 |

(continued)

| Compound | Structure | Molecular Weight (g.mol-1) |
|---|---|---|
| C26 | 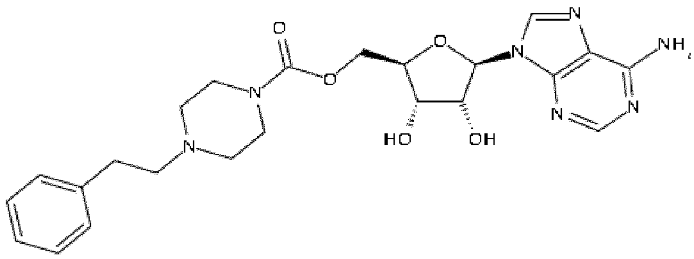 | 483.53 |
| C31 | 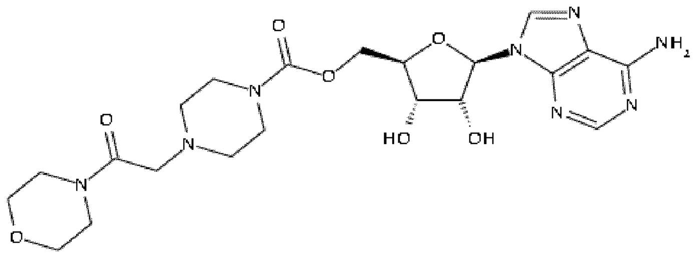 | 506.52 |
| C32 | 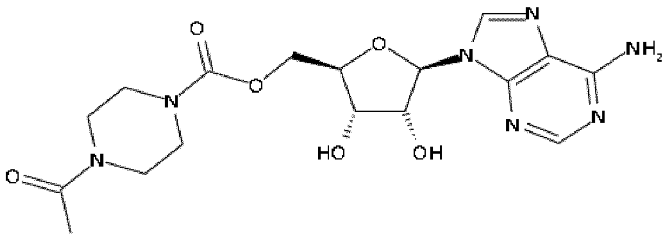 | 421.42 |
| C34 | 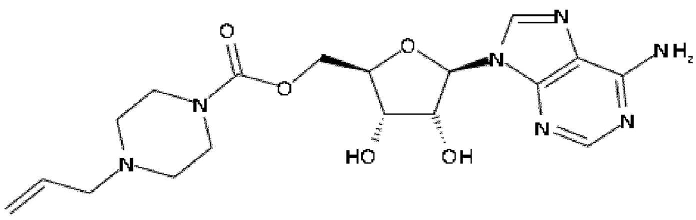 | 419.44 |
| C35 | 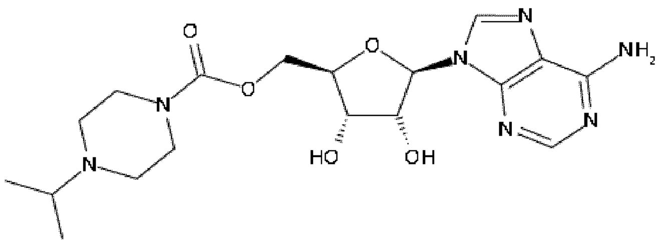 | 421.46 |
| C37 | 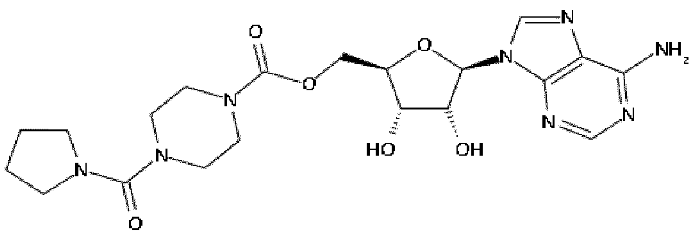 | 476.50 |

(continued)

| Compound | Structure | Molecular Weight (g.mol-1) |
|---|---|---|
| C39 | 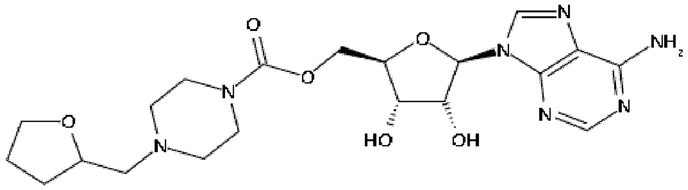 | 463.50 |
| C43 | 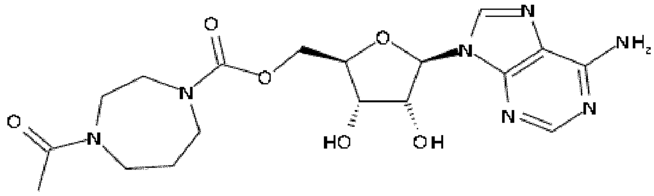 | 435.44 |
| C44 | 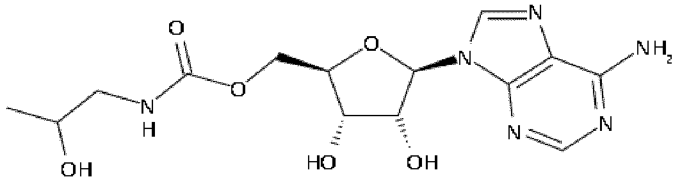 | 368.35 |
| C45 | 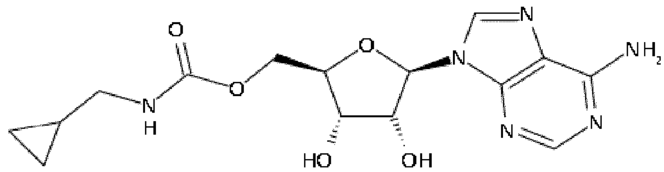 | 364.36 |
| C46 | 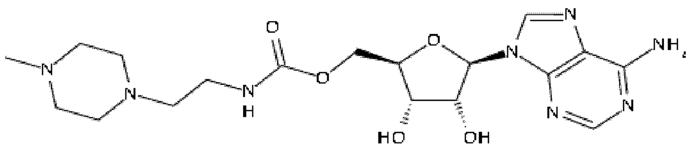 | 436.47 |
| C49 | 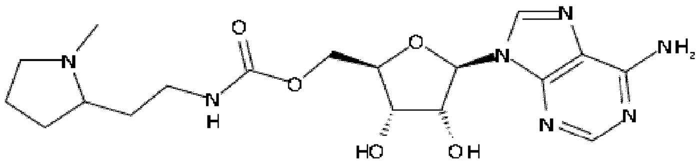 | 421.46 |
| C53 | 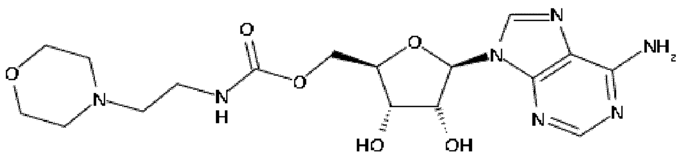 | 423.43 |
| C55 | 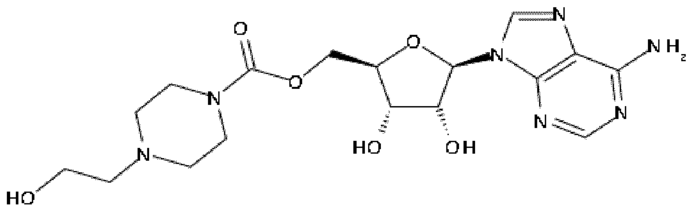 | 423.43 |

(continued)

| Compound | Structure | Molecular Weight (g.mol-1) |
|---|---|---|
| C56 | 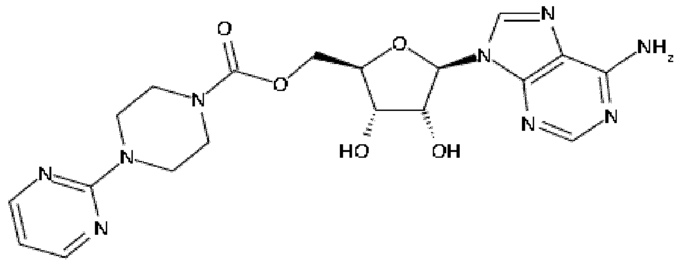 | 457.45 |
| C59 | 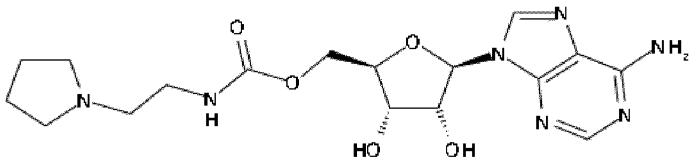 | 407.43 |
| C64 | 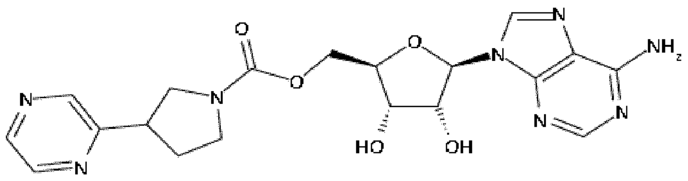 | 442.44 |
| C65 | 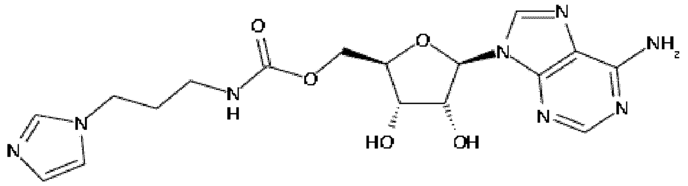 | 418.42 |
| C66 | 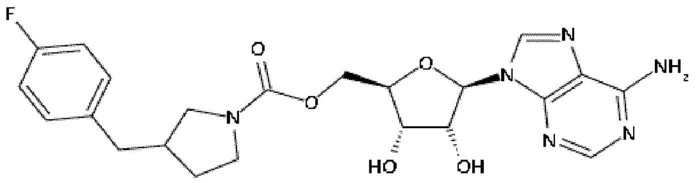 | 472.48 |
| C67 | 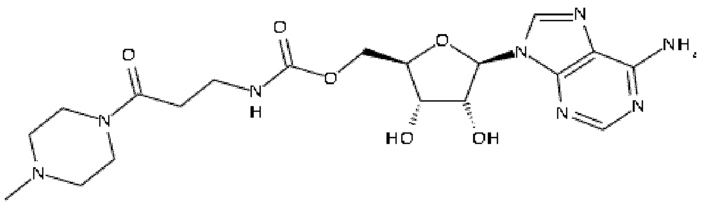 | 464.49 |
| C68 | 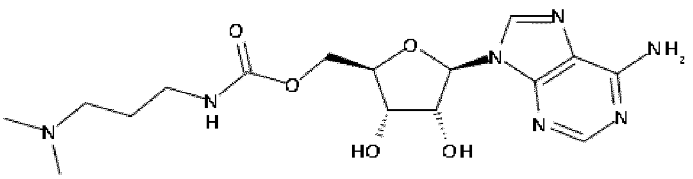 | 395.42 |

(continued)

| Compound | Structure | Molecular Weight (g.mol-1) |
|---|---|---|
| C70 | 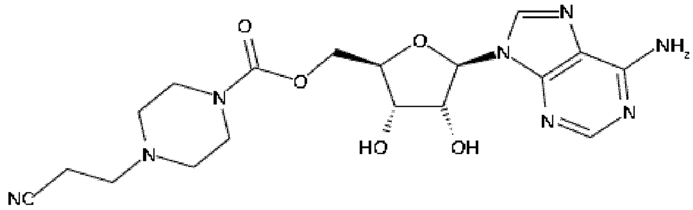 | 432.44 |
| C71 | 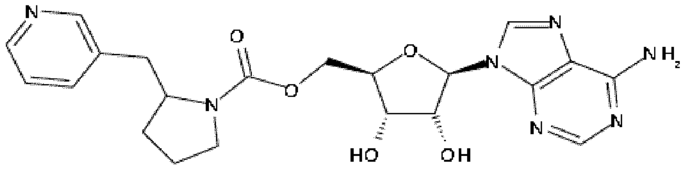 | 455.48 |
| C72 | 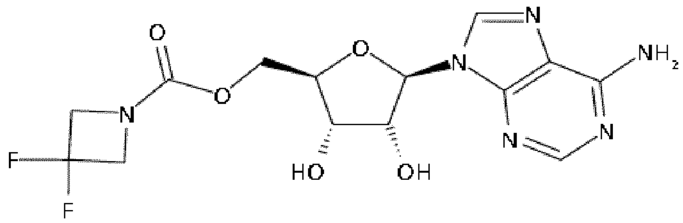 | 386.32 |
| C78 | 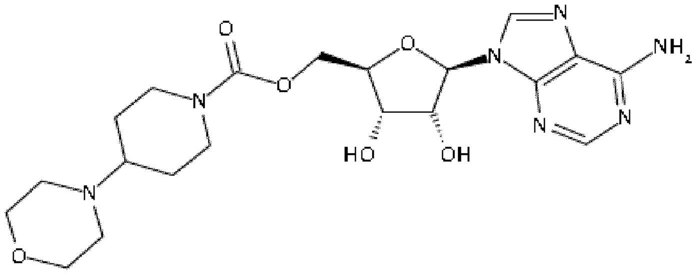 | 463.50 |
| C79 | 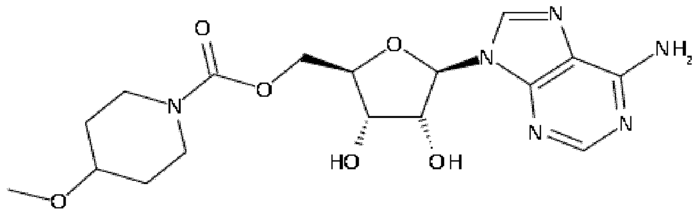 | 408.42 |
| C85 | 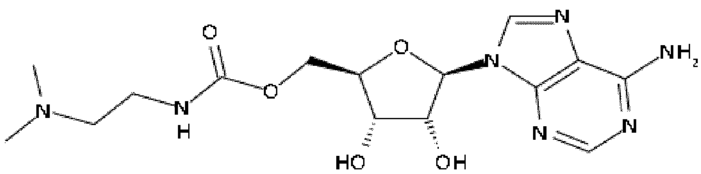 | 381.39 |
| C88 | 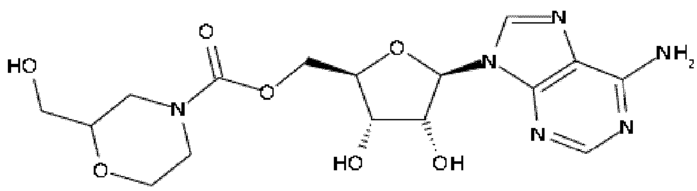 | 410.39 |

(continued)

| Compound | Structure | Molecular Weight (g.mol-1) |
|---|---|---|
| C89 | 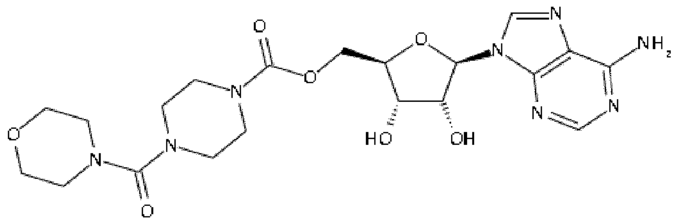 | 492.50 |
| C90 | 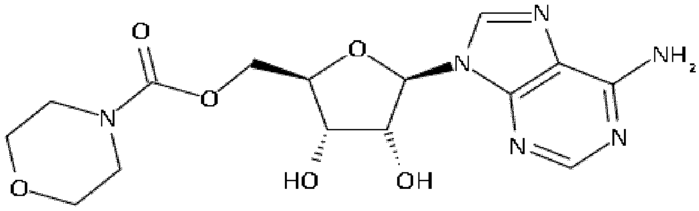 | 380.36 |
| C92 | 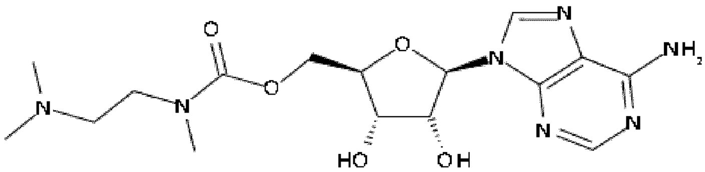 | 395.42 |
| C93 | 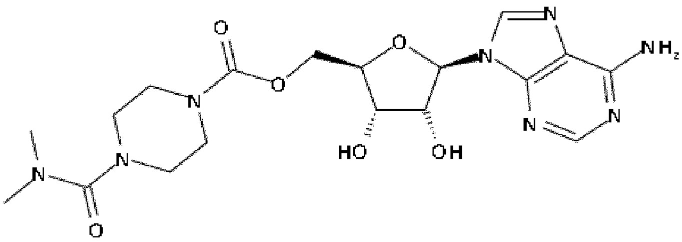 | 450.46 |
| C95 | 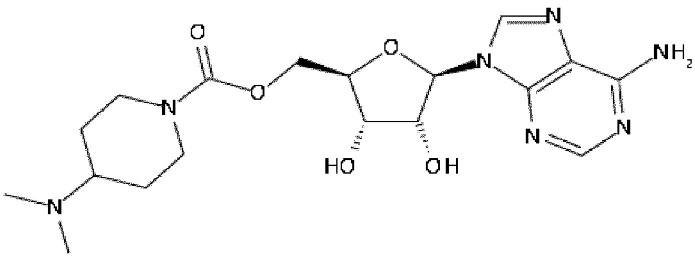 | 421.46 |
| C98 | 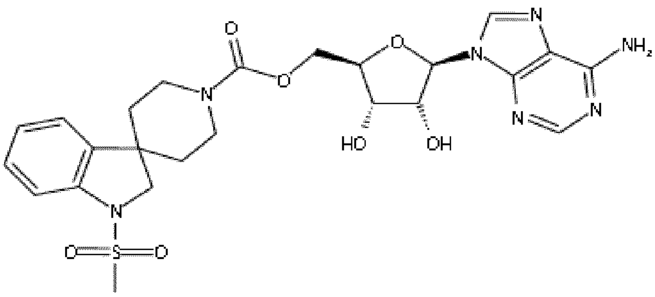 | 559.61 |

(continued)

| Compound | Structure | Molecular Weight (g.mol-1) |
|---|---|---|
| C100 | 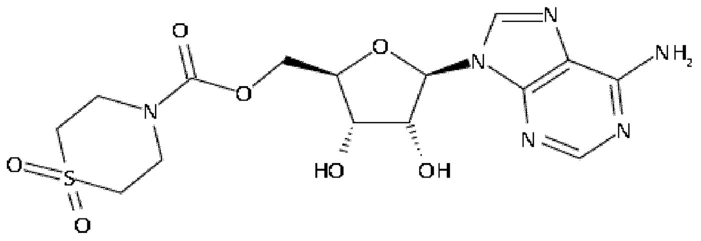 | 428.43 |

**Example 2: Biological assays measuring the protein kinase inhibition**

A biochemical assay was then performed in order to measure the protein kinase inhibition. It employed a fluorescence-based coupled-enzyme format and was based on the differential sensitivity of phosphorylated and non-phosphorylated peptides to proteolytic cleavage.

Peptide substrates were labeled with two fluorophores. The two fluorohores used were coumarin (donor) and fluorecein (acceptor), and each of the peptides was bearing one at one end, and one at the other end, which made up fluorescence resonance energy transfer (FRET) pair peptides.

In a primary reaction, the FRET-peptides were submitted to a phosphorylation reaction, in which one of adenosine triphosphate (ATP)'s phosphate groups was transferred to the peptide in the presence of kinase. When kinase inhibition takes place, the peptide will not be phosphorylated. In a secondary reaction, both the phosphorylated FRET-peptides and the non-phosphorylated FRET-peptides were reacted with a development reagent. It has been observed that non-phosphorylated FRET-peptides were cleaved, whereas the phosphorylted FRET-peptide remained intact.

Cleavage disrupts FRET between the donor (i.e., coumarin) and acceptor (i.e., fluorescein) fluorophores on the FRET-peptide, whereas uncleaved, phosphorylated FRET-peptides maintain FRET. A ratiometric method, which calculates the Emission Ratio of donor emission to acceptor emission after excitation of the donor fluorophore at 400 nm, is used to quantitate reaction progress, as shown in the equation below.

$$\text{Emission Ratio} = \frac{\text{Coumarin emission (445 nm)}}{\text{Fluorescein emission (520 nm)}}$$

A significant benefit of this ratiometric method for quantitating reaction progress is the elimination of well-to-well variations in FRET-peptide concentration and signal intensities. As a result, the assay yields very high Z'factor values (>0.7) at a low percent phosphorylation. Both cleaved and uncleaved FRET-peptides contribute to the fluorescence signals and therefore to the Emission Ratio. The extent of phosphorylation of the FRET-peptide can be calculated from the Emission Ratio. The Emission Ratio will remain low if the FRET-peptide is phosphorylated (i.e., no kinase inhibition) and will be high if the FRET-peptide is non-phosphorylated (i.e., kinase inhibition).

<u>Assay conditions</u>

The Test Compounds were screened in 1% DMSO. For 10 point titrations, 3-fold serial dilutions were prepared from the starting concentration.

All Peptide/Kinase Mixtures were diluted to a 2X working concentration in the appropriate Kinase Buffer (50 mM HEPES pH 7.5, 0.01% BRIJ-35, 10 mM $MgCl_2$, 1 mM EDTA).

All ATP Solutions were diluted to a 4X working concentration in Kinase Buffer. ATP Km apparent was previously determined using another assay.

The Development Reagent was diluted in Development Buffer. For 5 mL 10X Novel PKC Lipid Mix, 10X Novel PKC Lipid Mix being composed of 2 mg/ml Phosphatidyl Serine, 0.2 mg/ml DAG in 20 mM HEPES, pH 7.4, 0.3% CHAPS, 10 mg of Phosphatidyl Serine (Avanti Polar Lipids Part# 8400032C or 840039C) and 1 mg DAG (Avanti Polar Lipids Part# 800811C) were added to a glass tube.

The chloroform was removed from the lipid mixture by evaporating to a clear, thin film under a stream of nitrogen. Continuous rotation of the tube, at an angle to ensure maximum surface area of the lipid solution, has promoted the

thinnest film. 5mL resuspension buffer, 20 mM HEPES, 0.3% CHAPS, pH 7.4, were added to the dried lipid mix, which was then gently heated to 50-60°C for 1-2 minutes and vortexed in short intervals until the lipids are dissolved to a clear or slightly hazy solution. The lipids were typically in solution after 2-3 heat-vortex cycles. The solution was cooled to room temperature, aliquoted into single use volumes and stored at -20 °C.

Assay Protocol

A bar-coded Corning and low volume NBS, black 384-well plate (Corning Cat. #3676*)* were used.

2.5 μL - 4X Test Compound or 100 nL 100X plus 2.4 μL kinase buffer were mixed together with 2. 5 μL - 2X Peptide/Kinase Mixture and 3. 2.5 μL - 4X ATP Solution.

The solution was plate-shaked for 30 seconds, then incubated 60 minutes at room temperature for kinase reaction. 5 μL of Development Reagent Solution were then added, followed by a 30-second plate shake and a 60-minute Development Reaction incubation at room temperature. The results were read on a fluorescence plate reader and the data was analyzed.

Assay Controls

The following controls were made for each individual kinase and are located on the same plate as the kinase.

The maximum Emission Ratio was established by the 0% Phosphorylation Control (100% Inhibition Control), which contains no ATP and therefore has exhibited no kinase activity. This control yields 100% cleaved peptide in the Development Reaction.

The 100% Phosphorylation Control, which consists of a synthetically phosphorylated peptide of the same sequence as the peptide substrate, was designed to allow for the calculation of percent phosphorylation. This control yields a very low percentage of cleaved peptide in the Development Reaction. The 0% Phosphorylation and 100% Phosphorylation Controls allow to calculate the percent Phosphorylation achieved in a specific reaction well. Control wells did not include any kinase inhibitors.

The minimum Emission Ratio in a screen was established by the 0% Inhibition Control, which contains active kinase. This control was designed to produce a 10-50% phosphorylated peptide in the Kinase Reaction. Cascade assays may produce up to 70% phosphorylated peptide.

A known inhibitor control standard curve, 10 point titration, was run for each individual kinase on the same plate as the kinase to ensure the kinase was inhibited within an expected half maximal inhibitory concentration (IC50) range previously determined. The following controls were prepared for each concentration of Test Compound assayed.

The Development Reaction Interference was established by comparing the Test Compound Control wells that did not contain ATP versus the 0% Phosphorylation Control (which does not contain the Test Compound). The expected value for a non-interfering compound should be 100%. Any value outside of 90% to 110% was flagged.

The Test Compound Fluorescence Interference was determined by comparing the Test Compound Control wells that did not contain the Kinase/Peptide Mixture (zero peptide control) versus the 0% Inhibition Control. The expected value for a non-fluorescence compound should be 0%. Any value greater than 20% was flagged.

Results

The potency of the compound in inhibiting each protein kinase tested at a concentration of 10 μM is depicted in the table below. The compounds that show the highest Emission Ratio are the ones that also show the highest protein kinase inhibition properties.

Table 2

| Protein Kinase | C1 | C10 | C100 | C13 | C19 | C22 | C26 | C4 | C46 | C5 | C66 | C70 | C8 | C85 | C89 | C9 | C98 |
|---|---|---|---|---|---|---|---|---|---|---|---|---|---|---|---|---|---|
| ABL1 | 13 | 10 | 1 | 0 | -3 | -5 | 2 | 11 | 10 | 4 | -2 | -7 | 22 | -2 | -3 | -2 | 15 |
| ACVR1B (ALK4) | 2 | 4 | 3 | 7 | 6 | 3 | 0 | 1 | 6 | 7 | 1 | 1 | 0 | 0 | 0 | -5 | -1 |
| AKT1 (PKB alpha) | -2 | 4 | 8 | 2 | 3 | 0 | 0 | -2 | 4 | 1 | -1 | 8 | 7 | 4 | 2 | 3 | 2 |
| AMPK A1/B1/G1 | 17 | 11 | 4 | 6 | 5 | 4 | 3 | 17 | 4 | 2 | 14 | 8 | 17 | 4 | 2 | 4 | 9 |
| AURKA (Aurora A) | 9 | -1 | -7 | -1 | 6 | 5 | 1 | 8 | 3 | 3 | 4 | -1 | -7 | -11 | -7 | -5 | 0 |
| BTK | 9 | 7 | -5 | 9 | -1 | -1 | 0 | 2 | 0 | 0 | 11 | 1 | 4 | 0 | 4 | 9 | 16 |
| CDK1/cyclin B | -2 | 11 | 5 | 9 | 7 | 7 | 8 | 8 | 7 | 6 | 11 | 4 | 4 | 3 | 1 | 3 | 8 |
| CHEK1 (CHK1) | -20 | -3 | -8 | -8 | -16 | -14 | 20 | -11 | 2 | -9 | 18 | -11 | -8 | -9 | -20 | 14 | -8 |
| CSNK1G2 (CK1 gamma 2) | -2 | 7 | -1 | 11 | 6 | 2 | 0 | 7 | -4 | 5 | 10 | 0 | 13 | 0 | 0 | -5 | 15 |
| CSNK2A1 (CK2 alpha 1) | -5 | 2 | 5 | 3 | 5 | 2 | 3 | 8 | 5 | -1 | -5 | 4 | 5 | 7 | 8 | 5 | 2 |
| DYRK3 | 12 | 8 | 12 | 9 | 9 | 3 | 2 | 5 | 4 | 15 | 7 | 6 | 31 | 7 | 3 | 11 | 5 |
| EGFR (ErbB1) | 34 | 27 | 8 | 8 | 17 | 11 | 13 | 22 | 14 | 16 | 31 | -1 | 14 | 20 | 28 | -2 | 5 |
| EPHA2 | 8 | 9 | 4 | 3 | 7 | 1 | -1 | 4 | 3 | 3 | 9 | 4 | 12 | 4 | 6 | 5 | 5 |
| ERBB2 (HER2) | 5 | -5 | 2 | 3 | -7 | -9 | 8 | 2 | -14 | 5 | 11 | 0 | -7 | -13 | -7 | -19 | 5 |
| FGFR1 | 21 | 20 | 0 | 5 | 6 | 0 | 9 | 6 | 9 | -3 | 13 | 1 | 26 | 4 | 16 | 3 | 9 |
| FLT3 | 6 | 13 | 9 | 12 | 23 | 9 | 18 | 19 | 10 | 6 | 20 | 7 | 12 | 12 | 10 | 8 | 13 |

| | | | | | | | | | | | | | | | | | |
|---|---|---|---|---|---|---|---|---|---|---|---|---|---|---|---|---|---|
| FRAP1 (mTOR) | -2 | -3 | -2 | -1 | 5 | 0 | -6 | -5 | 5 | 2 | -4 | -6 | 5 | 2 | -4 | -3 | 2 |
| GSK3B (GSK3 beta) | 7 | 8 | 5 | 4 | 6 | 3 | 6 | 8 | 5 | 9 | 1 | 0 | 21 | -5 | -2 | -6 | -1 |
| IGF1R | 0 | 8 | 11 | 2 | -2 | 4 | 8 | 11 | 7 | 9 | 1 | 1 | 5 | 8 | 2 | 9 | 8 |
| IKBKB (IKK beta) | -10 | -11 | -9 | -7 | -6 | -13 | -8 | -6 | -3 | -2 | -11 | -12 | -6 | -10 | -10 | -8 | -8 |
| INSR | 0 | 3 | -2 | -3 | 0 | -5 | 2 | 5 | 0 | 4 | -5 | -2 | 2 | 3 | 0 | 4 | -2 |
| IRAK4 | -2 | 6 | 0 | 13 | -3 | 1 | 2 | 3 | 7 | 6 | -2 | 0 | -8 | -1 | -5 | -2 | -17 |
| JAK3 | -2 | -2 | -2 | 1 | -2 | -8 | -7 | 6 | 2 | 3 | 0 | -8 | -2 | -12 | 2 | -8 | -1 |
| KDR (VEGFR2) | -6 | 8 | -3 | -3 | -10 | -7 | -3 | 4 | -7 | -2 | 3 | -12 | 0 | 2 | -6 | -3 | 8 |
| KIT | 10 | 7 | -9 | 18 | -1 | 3 | 6 | 20 | 6 | -8 | -7 | 12 | 28 | 10 | -16 | -3 | 20 |
| LCK | 72 | 62 | 9 | 44 | 32 | 15 | 57 | 60 | 13 | 17 | 57 | 11 | 26 | 25 | 9 | 19 | 84 |
| MAP2K1 (MEK1) | 11 | 0 | 5 | 1 | -2 | -3 | 4 | 4 | -1 | 51 | -1 | 3 | 10 | -3 | -2 | -3 | 4 |
| MAP4K4 (HGK) | 17 | 8 | -6 | -5 | 17 | 17 | 17 | 16 | 13 | 17 | 1 | -5 | 9 | -8 | 9 | -2 | 68 |
| MAPK1 (ERK2) | 7 | 8 | 13 | 10 | 7 | 9 | 11 | 8 | 9 | 44 | 9 | 16 | 16 | 8 | 9 | 11 | 16 |
| MAPK14 (p38 alpha) | -3 | 6 | -4 | 0 | -1 | -1 | -6 | -1 | 5 | 0 | 18 | 4 | -3 | 0 | -2 | -4 | 20 |
| MAPK3 (ERK1) | 5 | 5 | 10 | 5 | 4 | 1 | 8 | 6 | 2 | 36 | -2 | 4 | 12 | 2 | 3 | 5 | 8 |
| MAPK8 (JNK1) | 12 | 6 | 7 | 2 | 0 | 5 | 8 | 14 | -2 | 8 | 6 | 8 | 13 | 4 | 5 | 3 | 4 |
| MARK2 | 3 | 8 | 15 | 11 | 7 | 8 | 18 | 20 | 4 | -2 | -4 | 6 | 10 | 4 | -3 | 6 | 2 |
| MET (cMet) | 0 | -1 | -3 | 0 | 2 | 1 | -5 | 3 | 8 | 11 | -14 | -2 | -16 | -12 | -4 | -11 | -12 |
| NEK1 | -4 | 11 | -3 | 4 | -2 | -2 | -7 | 4 | 7 | -2 | -5 | 6 | -5 | 1 | -7 | -3 | 0 |
| PAK4 | 15 | 14 | 2 | 7 | 3 | 7 | 6 | 12 | 4 | 3 | 10 | 4 | 19 | 1 | -4 | 1 | 7 |
| PDGFRB (PDGFR beta) | 1 | 7 | 4 | 6 | 6 | 4 | 1 | 5 | 1 | 0 | 4 | 4 | 5 | 8 | 9 | -1 | 10 |
| PHKG2 | -3 | 0 | 1 | 0 | -3 | -6 | -1 | -2 | 0 | -4 | -2 | 1 | -3 | 1 | -2 | -4 | -1 |
| PIM1 | -2 | 1 | -1 | 1 | -7 | -6 | -5 | -8 | 8 | -1 | 7 | 5 | 11 | 0 | -4 | 3 | 6 |
| PLK1 | -8 | -13 | -11 | -12 | -6 | -5 | -8 | -3 | -5 | -8 | -5 | 0 | -1 | -3 | -10 | -1 | -7 |
| PRKACA (PKA) | 21 | 10 | -3 | -1 | 4 | 0 | -1 | 20 | 3 | 7 | 1 | -4 | 43 | -2 | -1 | -6 | -2 |

| | | | | | | | | | | | | | | | | | |
|---|---|---|---|---|---|---|---|---|---|---|---|---|---|---|---|---|---|
| PRKCB1 (PKC beta I) | 6 | 5 | 1 | 7 | 4 | -8 | 0 | -1 | 0 | 9 | 1 | -5 | -11 | -1 | -6 | -6 | -1 |
| ROCK1 | 18 | -2 | -6 | -7 | 2 | -7 | -12 | -7 | -3 | -8 | -1 | -3 | 2 | -7 | -15 | -10 | -8 |
| RPS6KA3 (RSK2) | 8 | 8 | 1 | 3 | 1 | 2 | 0 | 9 | 6 | 9 | 6 | 2 | 19 | 7 | 0 | 1 | 4 |
| RPS6KB1 (p70S6K) | 15 | 5 | 13 | 10 | 3 | 0 | 8 | 22 | 14 | 12 | 16 | 6 | 11 | -1 | 0 | 3 | 0 |
| SRC | 46 | 25 | 8 | 29 | -1 | 12 | 29 | 33 | 17 | 15 | 38 | 8 | -13 | 17 | 8 | 16 | 58 |
| SYK | 3 | 1 | -3 | 0 | -1 | -8 | -4 | 4 | 0 | -4 | 1 | -1 | 7 | -1 | -5 | -4 | 2 |
| TEK (Tie2) | 3 | 6 | 2 | 5 | 6 | 0 | -4 | 12 | 0 | 2 | 4 | 1 | -3 | 0 | -2 | -4 | 58 |

| Legend |
|---|
| < 40% Inhibition |
| 40% - 80% Inhibition |
| ≥ 80% Inhibition |

**Claims**

1. An *in vitro* method of inhibiting protein kinase activity which comprises contacting a protein kinase with a compound of formula (II), or the N-oxide, pharmaceutically acceptable salt, pharmaceutically acceptable solvate, or stereoisomer thereof,

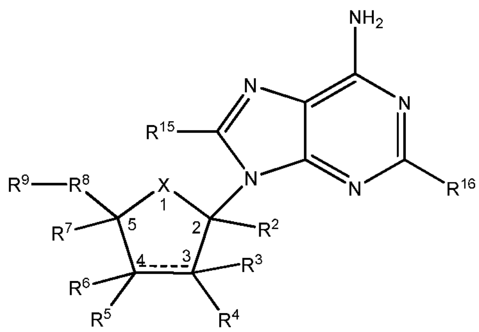

(II)

wherein

the dash bond (represented by - - - - - - ) represents an optional double bond; provided that when there is a double bond in the 5-membered ring, the substituents $\mathbf{R^3}$ and $\mathbf{R^6}$ are not present;
**X** is selected from O and S;
$\mathbf{R^2}$ is selected from hydrogen; Het; aryl; $C_{1\text{-}6}$alkyl optionally substituted with halo, hydroxy, or Het; halo$C_{1\text{-}6}$alkyl; $C_{2\text{-}6}$alkenyl; $C_{2\text{-}6}$alkynyl; - C(=O)$NR^{11}R^{12}$; and a cyclo$C_{3\text{-}7}$alkyl or Het which is formed with any one of the carbon atoms of the depicted cyclopentane ring except carbon atom 2;
$\mathbf{R^3}$, $\mathbf{R^4}$, $\mathbf{R^5}$, $\mathbf{R^6}$, and $\mathbf{R^7}$ are each independently selected from hydrogen; halo; cyano; oxo; -$NR^{11}R^{12}$; -$OR^{13}$; -C(=O)$OR^{13}$; -C(=O)$NR^{11}R^{12}$; $C_{1\text{-}6}$alkyl optionally substituted with one or two hydroxy, halo, cyano, $C_{3\text{-}7}$cycloalkyl, aryl, or Het; halo$C_{1\text{-}6}$alkyl; $C_{2\text{-}6}$alkenyl; $C_{2\text{-}6}$alkynyl; and -SH;

wherein $\mathbf{R^3}$ and $\mathbf{R^4}$ can form $=CH_2$ or $=CF_2$;
wherein $\mathbf{R^5}$ and $\mathbf{R^6}$ can form $=CH_2$ or $=CF_2$;
wherein $\mathbf{R^3}$ and $\mathbf{R^4}$ can form a $C_{3-7}$cycloalkyl, $C_{3-7}$cycloalkenyl, or a Het;
wherein $\mathbf{R^4}$ and $\mathbf{R^5}$ can form a $C_{3-7}$cycloalkyl, $C_{3-7}$cycloalkenyl, or a Het;
wherein $\mathbf{R^5}$ and $\mathbf{R^6}$ can form a $C_{3-7}$cycloalkyl, $C_{3-7}$cycloalkenyl, or a Het;
wherein $\mathbf{R^6}$ and $\mathbf{R^7}$ can form a $C_{3-7}$cycloalkyl, $C_{3-7}$cycloalkenyl, or a Het;
wherein $\mathbf{R^4}$ and $\mathbf{R^7}$ can form a $C_{3-7}$cycloalkyl optionally substituted with oxo; $C_{3-7}$cycloalkenyl; or a Het;

$\mathbf{R^8}$ is methanediyl;
$\mathbf{R^9}$ is $-O-C(=O)NR^{11}R^{12}$;
$\mathbf{R^{10}}$ is hydrogen, $C_{1-8}$alkyl, $-C(=O)C_{1-8}$alkyl, $-C(=O)-N(C_{1-8}alkyl)_2$, $-C(=O)-N(cycloC_{3-7}alkyl)$, or Het;
$\mathbf{R^{11}}$ and $\mathbf{R^{12}}$ each independently are hydrogen; $C_{1-6}$alkyl optionally substituted with one, two, or three substituents selected from oxo, $-OR^{13}$, $C_{3-7}$cycloalkyl, aryl, and Het; halo$C_{1-6}$alkyl; $C_{2-6}$alkenyl; $C_{2-6}$alkynyl; aryl; Het; $C_{3-7}$cycloalkyl optionally substituted with $C_{1-6}$alkyl; with the proviso that at least one $R^{11}$ or $R^{12}$ is not hydrogen; or $\mathbf{R^{11}}$ **and** $\mathbf{R^{12}}$ together with the nitrogen atom to which they are attached form a Het ring;
$\mathbf{R^{13}}$ is hydrogen; $C_{2-6}$alkenyl; $C_{2-6}$alkynyl; aryl; Het; mono- or di$C_{1-6}$alkylamino; $C_{3-7}$cycloalkyl optionally substituted with $C_{1-6}$alkyl; or $C_{1-6}$alkyl optionally substituted with one, two, or three substituents selected from hydroxy, cyano, $C_{1-4}$alkoxy, $-O-P(=O)(-OR^{14})(-OR^{14})$, $C_{3-7}$cycloalkyl, aryl, and Het;
$\mathbf{R^{14}}$ is hydrogen, $C_{1-8}$alkyl, or halo$C_{1-8}$alkyl;
$\mathbf{R^{15}}$ is hydrogen, halo, $C_{1-6}$alkyl, $C_{2-6}$alkenyl, $C_{2-6}$alkynyl, amino, or oxo; and
$\mathbf{R^{16}}$ is hydrogen, halo, $C_{1-6}$alkyl, $C_{2-6}$alkenyl, $C_{2-6}$alkynyl, amino, or oxo;
each **Het** is independently a saturated, partially unsaturated or completely unsaturated monocycle, bicycle, or tricycle having 3 to 12 carbon atoms and containing 1, 2, 3, or 4 heteroatoms each independently selected from nitrogen, oxygen, and sulfur, said heterocycle being optionally substituted with one, two, three, or four substituents each independently selected from halo; oxo; nitro; cyano; $C_{1-6}$alkyl optionally substituted with hydroxy, $C_{1-6}$alkoxy, cyano, amino, aryl, -C(=O)-morpholinyl, or pyridinyl; $C_{2-6}$alkenyl; $C_{2-6}$alkynyl; $-OR^{13}$; $-C(=O)R^{13}$; $-C(=O)OR^{13}$; $-S(=O)_2R^{13}$; amino; mono- or di$C_{1-6}$alkylamino; azido; mercapto; halo$C_{1-6}$alkyl; halo$C_{1-6}$alkoxy; $C_{1-6}$alkylthio, $C_{3-7}$cycloalkyl; aryl; pyrazinyl; pyrrolidinyl; piperidinyl; pyrimidinyl; thieno[2,3-d]pyrimidinyl; piperazinyl; and morpholinyl; and
each **aryl** as a group or part of a group is an optionally fused phenyl, naphthyl, or anthracenyl; the optional fusing occurring with one or two $C_{3-7}$cycloalkyls; wherein said aryl as a group is optionally substituted with one, two, or three substituents selected from halo, hydroxy, nitro, cyano, carboxyl, $C_{1-6}$alkyl, $C_{2-6}$alkoxy, $C_{1-6}$alkoxy$C_{1-6}$alkyl, $C_{1-6}$alkylcarbonyl, amino, mono- or di$C_{1-6}$alkylamino, azido, mercapto,$-S(=O)_2R^{13}$, halo$C_{1-6}$alkyl, halo$C_{1-6}$alkoxy, cyclopropyl, pyrrolidinyl, piperidinyl, piperazinyl, 4-$C_{1-6}$alkylpiperazinyl, 4-$C_{1-6}$alkylcarbonylpiperazinyl, and morpholinyl; and wherein the morpholinyl and piperidinyl groups may optionally substituted with one or two $C_{1-6}$alkyls.

**2.** The *in vitro* method of inhibiting protein kinase activity according to claim 1, wherein $\mathbf{R^2}$ is hydrogen; or $C_{1-6}$alkyl optionally substituted with halo or hydroxy.

**3.** The *in vitro* method of inhibiting protein kinase activity according to claim 1 or claim 2, wherein compound of formula (II) is a compound of formula (III)

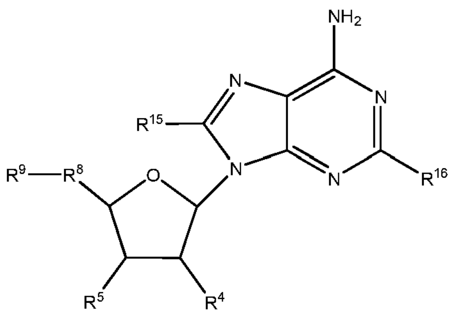

(III)

$\mathbf{R^4}$, $\mathbf{R^5}$, $\mathbf{R^8}$, and $\mathbf{R^9}$, $\mathbf{R^{15}}$, and $\mathbf{R^{16}}$ are as defined in claim 1.

**4.** The *in vitro* method of inhibiting protein kinase activity according to any one of claims 1 to 3, wherein compound of

formula (II) is a compound of formula (IV)

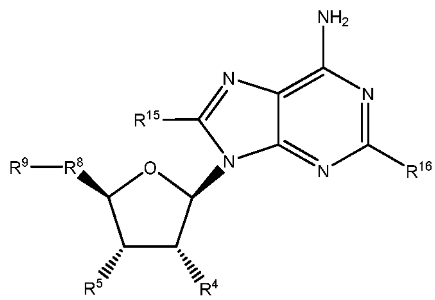

(IV)

$R^4$, $R^5$, $R^8$, and $R^9$, $R^{15}$, and $R^{16}$ are as defined in claim 1.

**5.** The *in vitro* method of inhibiting protein kinase activity according to any one of claims 1 to 3, wherein compound of formula (II) is a compound of formula (V)

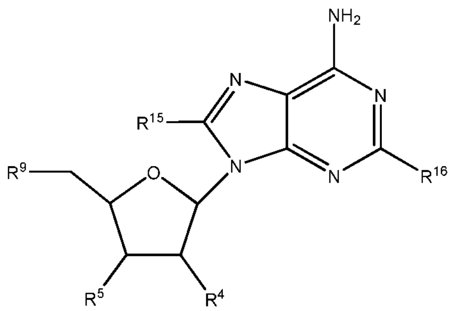

(V)

and $R^4$, $R^5$, and $R^9$ are as defined in claim 1, and $R^{15}$, and $R^{16}$ are as defined in claim 2.

**6.** The *in vitro* method of inhibiting protein kinase activity according to any one of claims 1 to 4, wherein compound of formula (II) is a compound of formula (VI)

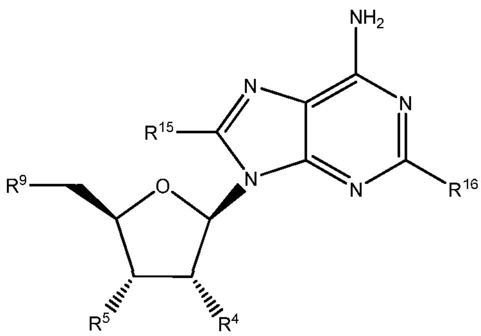

(VI)

and $R^4$, $R^5$, and $R^9$, $R^{15}$, and $R^{16}$ are as defined in claim 1.

**7.** A compound of formula (II), (III), (IV), (V), or (VI), as defined in claims 1 to 6, or the N-oxide, pharmaceutically acceptable salt, pharmaceutically acceptable solvate, or stereoisomer thereof;
with the proviso that the compound is not

5'-O-methyladenosine,
5'-O-n-butyladenosine,
2',5'-O-dimethyladenosine,
3',5'-O-dimethyladenosine,
2,5'-O-dimethyladenosine,

2-methyl-5'-O-n-butyladenosine,
$N^6$-5'-O-dimethyladenosine, or
2,$N^6$,5'-O-trimethyladenosine.

**8.** A pharmaceutical composition comprising a carrier, and as active ingredient an effective amount of a compound as defined in claim 7.

**9.** A compound according to claim 7, for use as a medicament.

**10.** A compound of formula (II), (III), (IV), (V), or (VI), as defined in claims 1 to 6, or the N-oxide, pharmaceutically acceptable salt, pharmaceutically acceptable solvate, or stereoisomer thereof for use in the treatment of a cardiovascular disease or for use in the treatment of a disease selected from cancer, metabolic diseases, rheumatoid arthritis, psoriasis, inflammatory bowel disease, Vogt-Koyanagi-Harada syndrome (noninfectious blindness), and Alzheimer; with the provisio that for the treatment of cardiovascular disease said compound is not 5'-O-methyladenosine, 5'-O-n-butyladenosine, 2',5'-O-dimethyladenosine, 3',5'-O-dimethyladenosine, 2,5'-O-dimethyladenosine, 2-methyl-5'-O-n-butyladenosine, $N^6$-5'-O-dimethyladenosine, or 2,$N^6$,5'-O-trimethyladenosine.

**Patentansprüche**

**1.** Ein *In-vitro*-Verfahren der Hemmung der Aktivität von Proteinkinase, welches das Inkontaktbringen einer Proteinkinase mit einer Verbindung der Formel (II), oder dem N-Oxid, pharmazeutisch akzeptablen Salz, pharmazeutisch akzeptablen Solvat, oder Stereoisomer davon, umfasst,

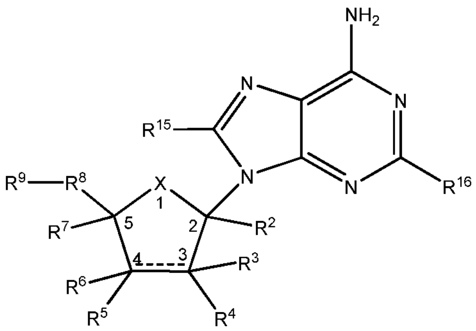

(II)

wobei

die Strich-Bindung (gezeigt durch - - - - - -) eine optionale Doppelbindung darstellt; vorausgesetzt, dass die Substituenten $\mathbf{R^3}$ und $\mathbf{R^6}$, wenn eine Doppelbindung im 5-gliedrigen Ring vorliegt, nicht vorhanden sind;
X aus O und S ausgewählt wird;
$\mathbf{R^2}$ ausgewählt wird aus Wasserstoff; Het; Aryl; $C_{1-6}$Alkyl optional ersetzt durch Halo, Hydroxy, oder Het; Halo$C_{1-6}$Alkyl; $C_{2-6}$Alkenyl; $C_{2-6}$Alkinyl; -C(=O)$NR^{11}R^{12}$; und einem Cyclo$C_{3-7}$Alkyl oder Het, geformt mit irgendeinem der Kohlenstoffatome des dargestellten Cyclopentanrings, ausgenommen Kohlenstoffatom 2;
$\mathbf{R^3}$, $\mathbf{R^4}$, $\mathbf{R^5}$, $\mathbf{R^6}$, und $\mathbf{R^7}$ jeweils unabhängig ausgewählt werden aus Wasserstoff; Halo; Cyano; Oxo; -$NR^{11}R^{12}$; -$OR^{13}$; -C(=O)$OR^{13}$; -C(=O)$NR^{11}R^{12}$; $C_{1-6}$Alkyl optional ersetzt durch ein oder zwei Hydroxy, Halo, Cyano, $C_{3-7}$Cycloalkyl, Aryl, oder Het; Halo$C_{1-6}$Alkyl; $C_{2-6}$Alkenyl; $C_{2-6}$Alkinyl; und -SH;

wobei $\mathbf{R^3}$ und $\mathbf{R^4}$ =$CH_2$ oder =$CF_2$ bilden können;
wobei $\mathbf{R^5}$ und $\mathbf{R^6}$ =$CH_2$ oder =$CF_2$ bilden können;
wobei $\mathbf{R^3}$ und $\mathbf{R^4}$ ein $C_{3-7}$Cycloalkyl, $C_{3-7}$Cycloalkenyl, oder einen Het, bilden können;
wobei $\mathbf{R^4}$ und $\mathbf{R^5}$ ein $C_{3-7}$Cycloalkyl, $C_{3-7}$Cycloalkenyl, oder einen Het, bilden können;
wobei $\mathbf{R^5}$ und $\mathbf{R^6}$ ein $C_{3-7}$Cycloalkyl, $C_{3-7}$Cycloalkenyl, oder einen Het, bilden können;
wobei $\mathbf{R^6}$ und $\mathbf{R^7}$ ein $C_{3-7}$Cycloalkyl, $C_{3-7}$Cycloalkenyl, oder einen Het, bilden können;
wobei $\mathbf{R^4}$ und $\mathbf{R^7}$ ein $C_{3-7}$Cycloalkyl optional ersetzt durch Oxo; $C_{3-7}$Cycloalkenyl; oder einen Het, bilden können;

$\mathbf{R^8}$ Methanediyl ist;

**$R^9$** -O-C(=O)$NR^{11}R^{12}$ ist;
**$R^{10}$** Wasserstoff, $C_{1-8}$Alkyl, -C(=O)$C_{1-8}$Alkyl, -C(=O)-N($C_{1-8}$Alkyl)$_2$, -C(=O)-N(Cyclo$C_{3-7}$Alkyl), oder Het, ist;
**$R^{11}$** und **$R^{12}$** jeweils unabhängig Wasserstoff; $C_{1-6}$Alkyl optional ersetzt durch einen, zwei oder drei Substituenten ausgewählt aus Oxo, -$OR^{13}$, $C_{3-7}$Cycloalkyl, Aryl, und Het; Halo$C_{1-6}$Alkyl; $C_{2-6}$Alkenyl; $C_{2-6}$Alkinyl; Aryl; Het; $C_{3-7}$Cycloalkyl optional ersetzt durch $C_{1-6}$Alkyl; sind; unter der Bedingung, dass zumindest ein $R^{11}$ oder $R^{12}$ nicht Wasserstoff ist; oder **$R^{11}$ und $R^{12}$** zusammen mit dem Stickstoffatom, an das sie gebunden sind, einen Het-Ring bilden;
**$R^{13}$** Wasserstoff; $C_{2-6}$Alkenyl; $C_{2-6}$Alkinyl; Aryl; Het; Mono- oder Di$C_{1-6}$Alkylamino; $C_{3-7}$Cycloalkyl optional ersetzt durch $C_{1-6}$Alkyl; oder $C_{1-6}$Alkyl optional ersetzt durch einen, zwei oder drei Substituenten ausgewählt aus Hydroxy, Cyano, $C_{1-4}$Alkoxy, -O-P(=O)(-$OR^{14}$)(-$OR^{14}$), $C_{3-7}$Cycloalkyl, Aryl, und Het; ist;
**$R^{14}$** Wasserstoff, $C_{1-8}$Alkyl, oder Halo$C_{1-8}$Alkyl ist;
**$R^{15}$** Wasserstoff, Halo, $C_{1-6}$Alkyl, $C_{2-6}$Alkenyl, $C_{2-6}$Alkinyl, Amino, oder Oxo ist; und
**$R^{16}$** Wasserstoff, Halo, $C_{1-6}$Alkyl, $C_{2-6}$Alkenyl, $C_{2-6}$Alkinyl, Amino, oder Oxo ist; jeder **Het** unabhängig ein gesättigter, teilweise ungesättigter oder völlig ungesättigter Monocyclus, Bicyclus, oder Tricyclus mit 3 bis 12 Kohlenstoffatomen ist und 1, 2, 3, oder 4 Heteroatome enthält, jeweils unabhängig ausgewählt aus Stickstoff, Sauerstoff, und Schwefel, wobei der erwähnte Heterocylus optional ersetzt wird durch einen, zwei, drei, oder vier Substituenten, die jeweils unabhängig ausgewählt werden aus Halo; Oxo; Nitro; Cyano; $C_{1-6}$Alkyl optional ersetzt durch Hydroxy, $C_{1-6}$Alkoxy, Cyano, Amino, Aryl, -C(=O)-Morpholinyl, oder Pyridinyl; $C_{2-6}$Alkenyl; $C_{2-6}$Alkinyl; -$OR^{13}$; -C(=O)$R^{13}$; - C(=O)$OR^{13}$; -S(=O)$_2R^{13}$; Amino; Mono- oder Di$C_{1-6}$Alkylamino; Azido; Mercapto; Halo$C_{1-6}$Alkyl; Halo$C_{1-6}$Alkoxy; $C_{1-6}$Alkylthio, $C_{3-7}$Cycloalkyl; Aryl; Pyrazinyl; Pyrrolidinyl; Piperidinyl; Pyrimidinyl; Thieno[2,3-d]Pyrimidinyl; Piperazinyl; und Morpholinyl; und
jedes **Aryl** als eine Gruppe oder Teil einer Gruppe ein optional anelliertes Phenyl, Naphthyl, oder Anthracenyl ist; wobei die optionale Anellierung mit einem oder zwei $C_{3-7}$Cycloalkylen stattfindet; wobei das erwähnte Aryl als eine Gruppe optional ersetzt wird durch einen, zwei oder drei Substituenten ausgewählt aus Halo, Hydroxy, Nitro, Cyano, Carboxyl, $C_{1-6}$Alkyl, $C_{2-6}$Alkoxy, $C_{1-6}$Alkoxy$C_{1-6}$Alkyl, $C_{1-6}$Alkylcarbonyl, Amino, Mono- oder Di$C_{1-6}$Alkylamino, Azido, Mercapto, -S(=O)$_2R^{13}$, Halo$C_{1-6}$Alkyl, Halo$C_{1-6}$Alkoxy, Cyclopropyl, Pyrrolidinyl, Piperidinyl, Piperazinyl, 4-$C_{1-6}$Alkylpiperazinyl, 4-$C_{1-6}$Alkyl-carbonylpiperazinyl, und Morpholinyl; und wobei die Morpholinyl- und Piperidinylgruppen optional durch ein oder zwei $C_{1-6}$Alkyle ersetzt werden können.

**2.** Das *In-vitro*-Verfahren der Hemmung der Aktivität von Proteinkinase nach Anspruch 1, wobei
**$R^2$** Wasserstoff; oder $C_{1-6}$Alkyl optional ersetzt durch Halo oder Hydroxy; ist.

**3.** Das *In-vitro*-Verfahren der Hemmung der Aktivität von Proteinkinase nach Anspruch 1 oder Anspruch 2, wobei die Verbindung nach Formel (II) eine Verbindung nach Formel (III) ist

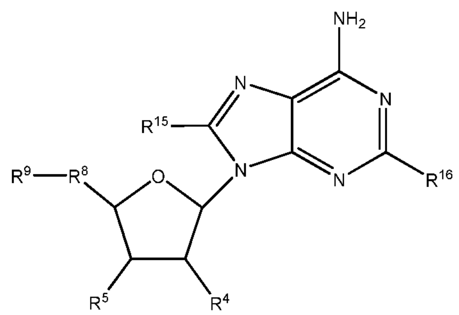

(III)

**$R^4$**, **$R^5$**, **$R^8$**, und **$R^9$, $R^{15}$**, und **$R^{16}$** wie definiert in Anspruch 1 sind.

**4.** Das *In-vitro*-Verfahren der Hemmung der Aktivität von Proteinkinase nach irgendeinem der Ansprüche 1 bis 3, wobei die Verbindung nach Formel (II) eine Verbindung nach Formel (IV) ist

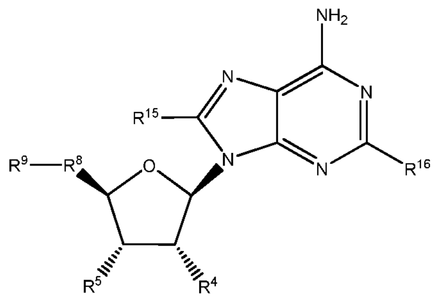

(IV)

$R^4$, $R^5$, $R^8$, und $R^9$, $R^{15}$, und $R^{16}$ wie definiert in Anspruch 1 sind.

**5.** Das *In-vitro*-Verfahren der Hemmung der Aktivität von Proteinkinase nach irgendeinem der Ansprüche 1 bis 3, wobei die Verbindung nach Formel (II) eine Verbindung nach Formel (V) ist

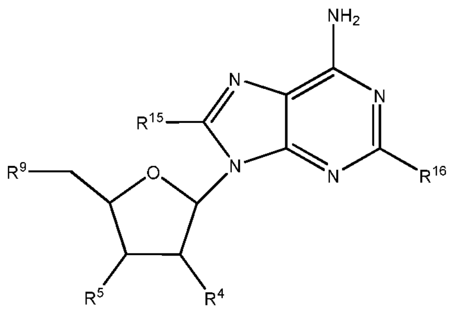

(V)

und $R^4$, $R^5$, und $R^9$ wie definiert in Anspruch 1 sind, und $R^{15}$, und $R^{16}$ wie definiert in Anspruch 2 sind.

**6.** Das *In-vitro*-Verfahren der Hemmung der Aktivität von Proteinkinase nach irgendeinem der Ansprüche 1 bis 4, wobei die Verbindung nach Formel (II) eine Verbindung nach Formel (VI) ist

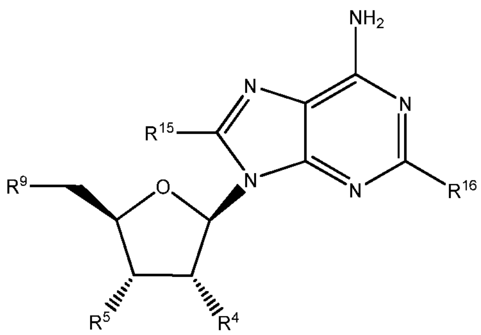

(VI)

und $R^4$, $R^5$, und $R^9$, $R^{15}$, und $R^{16}$ wie definiert in Anspruch 1 sind.

**7.** Eine Verbindung nach Formel (II), (III), (IV), (V), oder (VI), wie definiert in Ansprüchen 1 bis 6, oder das N-Oxid, pharmazeutisch akzeptable Salz, pharmazeutisch akzeptable Solvat, oder Stereoisomer davon; unter der Bedingung, dass die Verbindung nicht

5'-O-Methyladenosin,
5'-O-n-Butyladenosin,
2',5'-O-Dimethyladenosin,
3',5'-O-Dimethyladenosin,
2,5'-O-Dimethyladenosin,
2-Methyl-5'-O-n-Butyladenosin,
$N^6$-5'-O-Dimethyladenosin, oder

2,N$^6$ ,5'-O-Trimethyladenosin ist.

**8.** Eine pharmazeutische Zusammensetzung, welche einen Träger, und als Wirkstoff eine wirksame Menge einer Verbindung wie definiert in Anspruch 7 umfasst.

**9.** Eine Verbindung nach Anspruch 7, zur Anwendung als Arzneimittel.

**10.** Eine Verbindung nach Formel (II), (III), (IV), (V), oder (VI), wie definiert in Ansprüchen 1 bis 6, oder das N-Oxid, pharmazeutisch akzeptable Salz, pharmazeutisch akzeptable Solvat, oder Stereoisomer davon zur Anwendung in der Behandlung einer kardiovaskulären Erkrankung oder zur Anwendung in der Behandlung einer Erkrankung ausgewählt aus Krebs, Stoffwechselkrankheiten, rheumatoider Arthritis, Psoriasis, entzündlicher Darmerkrankung, Vogt-Koyanagi-Harada-Syndrom (nicht-infektiöse Blindheit), und Alzheimer; unter der Bedingung, dass für die Behandlung einer kardiovaskulären Erkrankung die erwähnte Verbindung nicht 5'-O-Methyladenosin, 5'-O-n-Butyladenosin, 2',5'-O-Dimethyladenosin, 3',5'-O-Dimethyladenosin, 2,5'-O-Dimethyladenosin, 2-Methyl-5'-O-n-Butyladenosin, N$^6$-5'-O-Dimethyladenosin, oder 2,N$^6$,5'-O-Trimethyladenosin ist.

## Revendications

**1.** Procédé *in vitro* d'inhibition de l'activité de la protéine kinase qui comprend la mise en contact d'une protéine kinase avec un composé de formule (II), ou le N-oxyde, le sel pharmaceutiquement acceptable, le solvate pharmaceutiquement acceptable, ou le stéréoisomère de celui-ci,

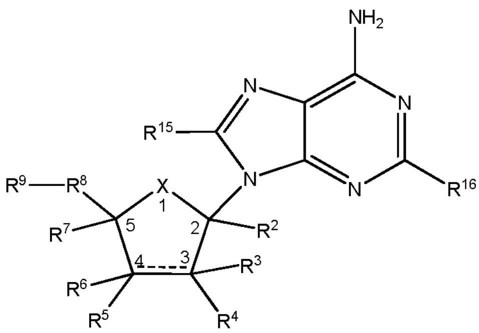

(II)

où

la liaison en pointillés (représentée par - - - - - -) représente une double liaison facultative; à condition que, lorsqu'il y a une double liaison dans le cycle à 5 chaînons, les substituants **R$^3$** et **R$^6$** ne soient pas présents ;
X est choisi parmi O, et S ;
**R$^2$** est choisi parmi l'hydrogène ; Het; un aryle ; un alkyle en $C_{1-6}$ éventuellement substitué par un halogène, un hydroxy ou Het ; un halogénoalkyle en $C_{1-6}$ ; un alcényle en $C_{2-6}$ ; un alcynyle en $C_{2-6}$; -C(=O)NR$^{11}$R$^{12}$ ; et un cycloalkyle en $C_{3-7}$ ou Het qui est formé avec l'un quelconque des atomes de carbone du cycle cyclopentane représenté à l'exception de l'atome de carbone 2 ;
**R$^3$**, **R$^4$**, **R$^5$**, **R$^6$** et **R$^7$** sont chacun indépendamment choisis parmi l'hydrogène; un halogène; un cyano ; un oxo ; -NR$^{11}$R$^{12}$ ; -OR$^{13}$; -C(=O)OR$^{13}$; -C(=O)NR$^{11}$R$^{12}$ ; un alkyle en $C_{1-6}$ éventuellement substitué par un ou deux hydroxy, halogène, cyano, cycloalkyle en $C_{3-7}$, un aryle ou Het ; un halogénoalkyle en $C_{1-6;}$ un alcényle en $C_{2-6}$; un alcynyle en $C_{2-6}$ ; et -SH ;
où **R$^3$** et **R$^4$** peuvent former =$CH_2$ ou =$CF_2$ ;
où **R$^5$** et **R$^6$** peuvent former =$CH_2$ ou =$CF_2$ ;
où **R$^3$** et **R$^4$** peuvent former un cycloalkyle en $C_{3-7}$, un cycloalcényle en $C_{3-7}$, ou un Het ;
où **R$^4$** et **R$^5$** peuvent former un cycloalkyle en $C_{3-7}$, un cycloalcényle en $C_{3-7}$ ou un Het ;
où **R$^5$** et **R$^6$** peuvent former un cycloalkyle en $C_{3-7}$, un cycloalcényle en $C_{3-7}$ ou un Het ;
où **R$^6$** et **R$^7$** peuvent former un cycloalkyle en $C_{3-7}$, un cycloalcényle en $C_{3-7}$ ou un Het ;
où **R$^4$** et **R$^7$** peuvent former un cycloalkyle en $C_{3-7}$ éventuellement substitué par un oxo ;

un cycloalcényle en $C_{3-7;}$ ou un Het ;

**$R^8$** est un méthanediyle ;

**$R^9$** est -O-C(=O)$NR^{11}R^{12}$ ;

**$R^{10}$** est un hydrogène, un alkyle en $C_{1-8}$, -C(=O)-alkyle en $C_{1-8}$, -C(=O)-N(alkyle en $C_{1-8}$)$_2$, -C(=O)-N(cycloalkyle en $C_{3-7}$), ou Het ;

**$R^{11}$** et **$R^{12}$** sont chacun indépendamment un hydrogène ; un alkyle en $C_{1-6}$ éventuellement substitué par un, deux ou trois substituants choisis parmi oxo, -$OR^{13}$, cycloalkyle en $C_{3-7}$, aryle et Het ; halogénoalkyle en $C_{1-6}$ ; alcényle en $C_{2-6}$ ; alcynyle en $C_{2-6}$ ; aryle ; Het ; cycloalkyle en $C_{3-7}$ éventuellement substitué par un alkyle en $C_{1-6}$; à condition qu'au moins un $R^{11}$ ou $R^{12}$ ne soit pas un'hydrogène ; ou **$R^{11}$ et $R^{12}$,** conjointement avec l'atome d'azote auquel ils sont liés, forment un cycle Het ;

**$R^{13}$** est un hydrogène ; un alcényle en $C_{2-6}$ ; un alcynyle en $C_{2-6}$ ; un aryle ; Het ; un monoou dialkylamino en $C_{1-6}$ ; un cycloalkyle en $C_{3-7}$ éventuellement substitué par un alkyle en $C_{1-6}$ ; ou un alkyle en $C_{1-6}$éventuellement substitué par un, deux ou trois substituants choisis parmi un hydroxy, un cyano, un alcoxy en $C_{1-4}$, -O-P(=O)(-$OR^{14}$)(-$OR^{14}$), un cycloalkyle en $C_{3-7}$, un aryle et Het ;

**$R^{14}$** est un hydrogène, un alkyle en $C_{1-8}$, ou un halogénoalkyle en $C_{1-8}$ ;

**$R^{15}$** est un hydrogène, un halogène, un alkyle en $C_{1-6}$, un alcényle en $C_{2-6}$, un alcynyle en $C_{2-6}$, un amino ou un oxo ; et

**$R^{16}$** est un hydrogène, un halogène, un alkyle en $C_{1-6}$, un alcényle en $C_{2-6}$, un alcynyle en $C_{2-6}$, un amino ou un oxo ;

chaque Het est indépendamment un monocycle, un bicycle ou un tricycle saturé, partiellement insaturé ou complètement insaturé ayant 3 à 12 atomes de carbone et contenant 1, 2, 3 ou 4 hétéroatomes choisis chacun indépendamment parmi l'azote, l'oxygène et le soufre, ledit hétérocycle étant éventuellement substitué par un, deux, trois ou quatre substituants choisis chacun indépendamment parmi un halogène ; oxo ; nitro ; cyano ; un alkyle en $C_{1-6}$ éventuellement substitué par un hydroxy, un alcoxy en $C_{1-6}$, un cyano, un amino, un aryle, une - C(=O)-morpholinyle ou une pyridinyle ; un alcényle en $C_{2-6}$; un alcynyle en $C_{2-6}$; -$OR^{13}$; - C(=O)$R^{13}$ ; -C(=O)$OR^{13}$ ; -S(=O)$_2R^{13}$ ; un amino ; mono- ou dialkylamino en $C_{1-6}$ ; azido ; mercapto ; halogénoalkyle en $C_{1-6}$ ; halogénoalcoxy en $C_{1-6}$; alkylthio en $C_{1-6}$ ; cycloalkyle en $C_{3-7}$ ; aryle ; pyrazinyle ; pyrrolidinyle ; pipéridinyle ; pyrimidinyle ; thiéno[2,3-d]pyrimidinyle ; pipérazinyle ; et morpholinyle ; et

chaque **aryle** en tant que groupe ou partie d'un groupe est un phényle, un naphtyle ou un anthracényle éventuellement fusionné ; la fusion éventuelle se produisant avec un ou deux cycloalkyles en $C_{3-7}$ ; où ledit aryle en tant que groupe est éventuellement substitué par un, deux ou trois substituants choisis parmi les groupes halo, hydroxy, nitro, cyano, carboxyle, alkyle en $C_{1-6}$, alcoxy en $C_{2-6}$, alcoxyalkyle l'alcocy étant en $C_{1-6}$ et l'alkyle en $C_{1-6}$, alkylcarbonyle en $C_{1-6}$, amino, mono- ou dialkylamino en $C_{1-6}$, azido, mercapto, -S(=O)$_2R^{13}$, halogénoalkyle en $C_{1-6}$, halogénoalcoxy en $C_{1-6}$, cyclopropyle, pyrrolidinyle, pipéridinyle, pipérazinyle, 4-alkylpipérazinyle, l'alkyle étant en $C_{1-6}$, 4-alkylcarbonylpipérazinyle, l'alkyle étant en $C_{1-6}$, et morpholinyle ; et où les groupes morpholinyle et pipéridinyle peuvent éventuellement être substitués par un ou deux alkyles en $C_{1-6}$.

**2.** Procédé *in vitro* d'inhibition de l'activité de la protéine kinase selon la revendication 1, dans lequel **$R^2$** est un hydrogène ; ou un alkyle en $C_{1-6}$ éventuellement substitué par un halogène ou un hydroxy.

**3.** Procédé *in vitro* d'inhibition de l'activité de la protéine kinase selon la revendication 1 ou la revendication 2, dans lequel le composé de formule (II) est un composé de formule (III)

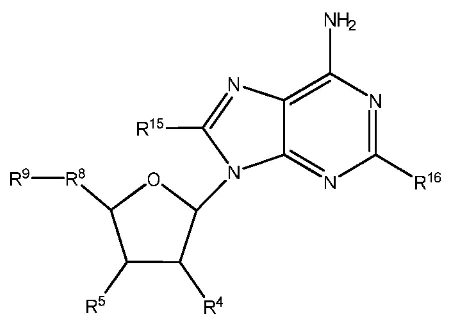

(III)

**$R^4$**, **$R^5$**, **$R^8$**, et **$R^9$, $R^{15}$**, et **$R^{16}$** sont tels que définis dans la revendication 1.

**4.** Procédé *in vitro* d'inhibition de l'activité de la protéine kinase selon l'une quelconque des revendications 1 à 3, dans lequel le composé de formule (II) est un composé de formule (IV)

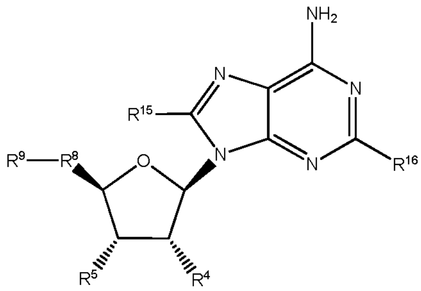

(IV)

**R⁴**, **R⁵**, **R⁸**, et **R⁹**, **R¹⁵**, et **R¹⁶** sont tels que définis dans la revendication 1.

**5.** Procédé *in vitro* d'inhibition de l'activité de la protéine kinase selon l'une quelconque des revendications 1 à 3, dans lequel le composé de formule (II) est un composé de formule (V)

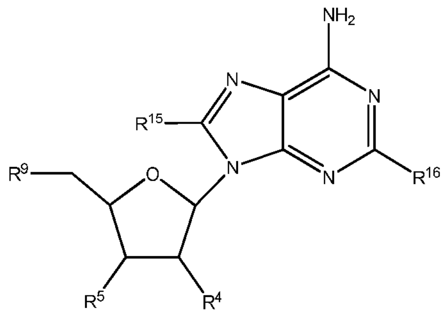

(V)

et **R⁴**, **R⁵**, et **R⁹** sont tels que définis dans la revendication 1, et **R¹⁵**, et **R¹⁶** sont tels que définis dans la revendication 2.

**6.** Procédé *in vitro* d'inhibition de l'activité de la protéine kinase selon l'une quelconque des revendications 1 à 4, dans lequel le composé de formule (II) est un composé de formule (VI)

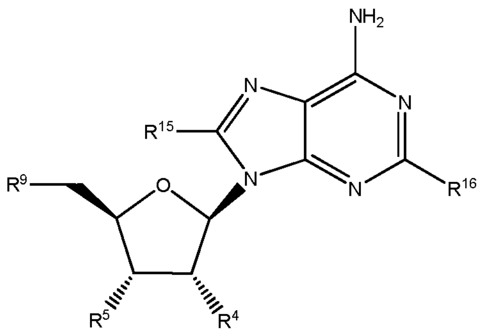

(VI)

et **R⁴**, **R⁵**, et **R⁹**, **R¹⁵**, et **R¹⁶** sont tels que définis dans la revendication 1.

**7.** Composé de formule (II), (III), (IV), (V), ou (VI), tel que défini dans les revendications 1 à 6, ou le N-oxyde, le sel pharmaceutiquement acceptable, le solvate pharmaceutiquement acceptable, ou le stéréoisomère de celui-ci ;.

à condition que le composé ne soit pas
5'-O-méthyladénosi ne,
5'-O-n-butyladénosi ne,
2',5'-O-diméthyladénosine,
la 3',5'-O-diméthyladénosine,
2,5'-O-diméthyladénosine,
2-méthyl-5'-O-n-butyladénosine,
$N^6$-5'-O-diméthyladénosine, ou

2,$N^6$,5'-O-triméthyladénosine.

**8.** Une composition pharmaceutique comprenant un vecteur et, en tant qu'ingrédient actif, une quantité efficace d'un composé tel que défini dans la revendication 7.

**9.** Composé selon la revendication 7, pour une utilisation comme médicament.

**10.** Composé de formule (II), (III), (IV), (V) ou (VI), tel que défini dans les revendications 1 à 6, ou le N-oxyde, le sel pharmaceutiquement acceptable, le solvate pharmaceutiquement acceptable ou le stéréoisomère de celui-ci pour une utilisation dans le traitement d'une maladie cardiovasculaire ou pour une utilisation dans le traitement d'une maladie choisie parmi le cancer, les maladies métaboliques, la polyarthrite rhumatoïde, le psoriasis, la maladie intestinale inflammatoire, le syndrome de Vogt-Koyanagi-Harada (cécité non infectieuse) et Alzheimer; à condition que, pour le traitement des maladies cardiovasculaires, ledit composé ne soit pas la 5'-O-méthyladénosine, la 5'-O-n-butyladénosine, la 2',5'-O-diméthyladénosine, la 3',5'-O-diméthyladénosine, la 2,5'-O-diméthyladénosine, la 2-méthyl-5'-O-n-butyladénosine, la $N^{6}$-5'-O-diméthyladénosine ou la 2,$N^6$,5'-O-triméthyladénosine.

*Fig. 1*

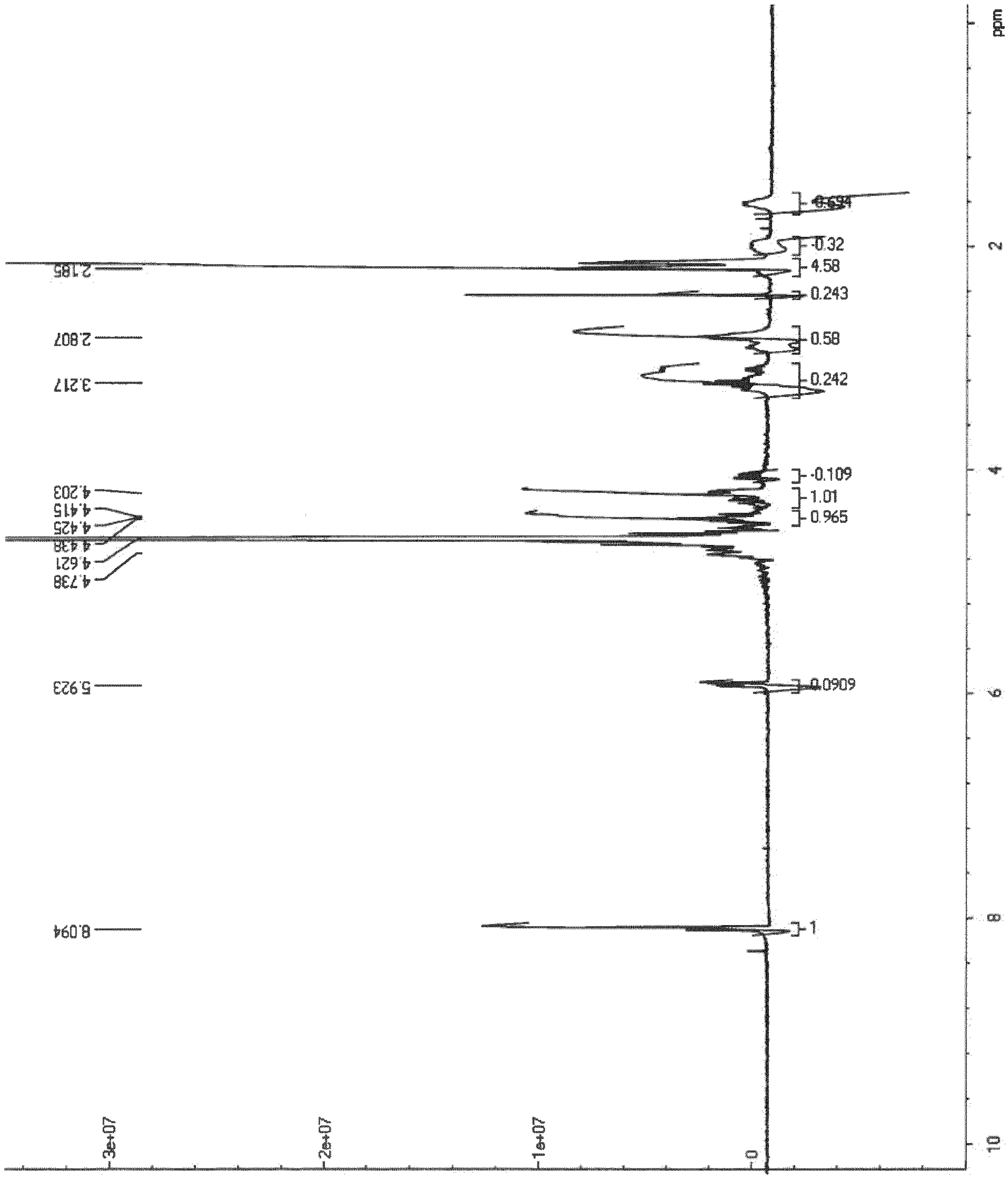
ppm
2
4
6
8
10
2.185
2.807
3.217
4.203
4.415
4.425
4.438
4.621
4.738
5.923
8.094
-0.32
4.58
0.243
0.58
0.242
-0.109
1.01
0.965
0.0909
1
-3e+07
-2e+07
-1e+07
0

Fig. 2

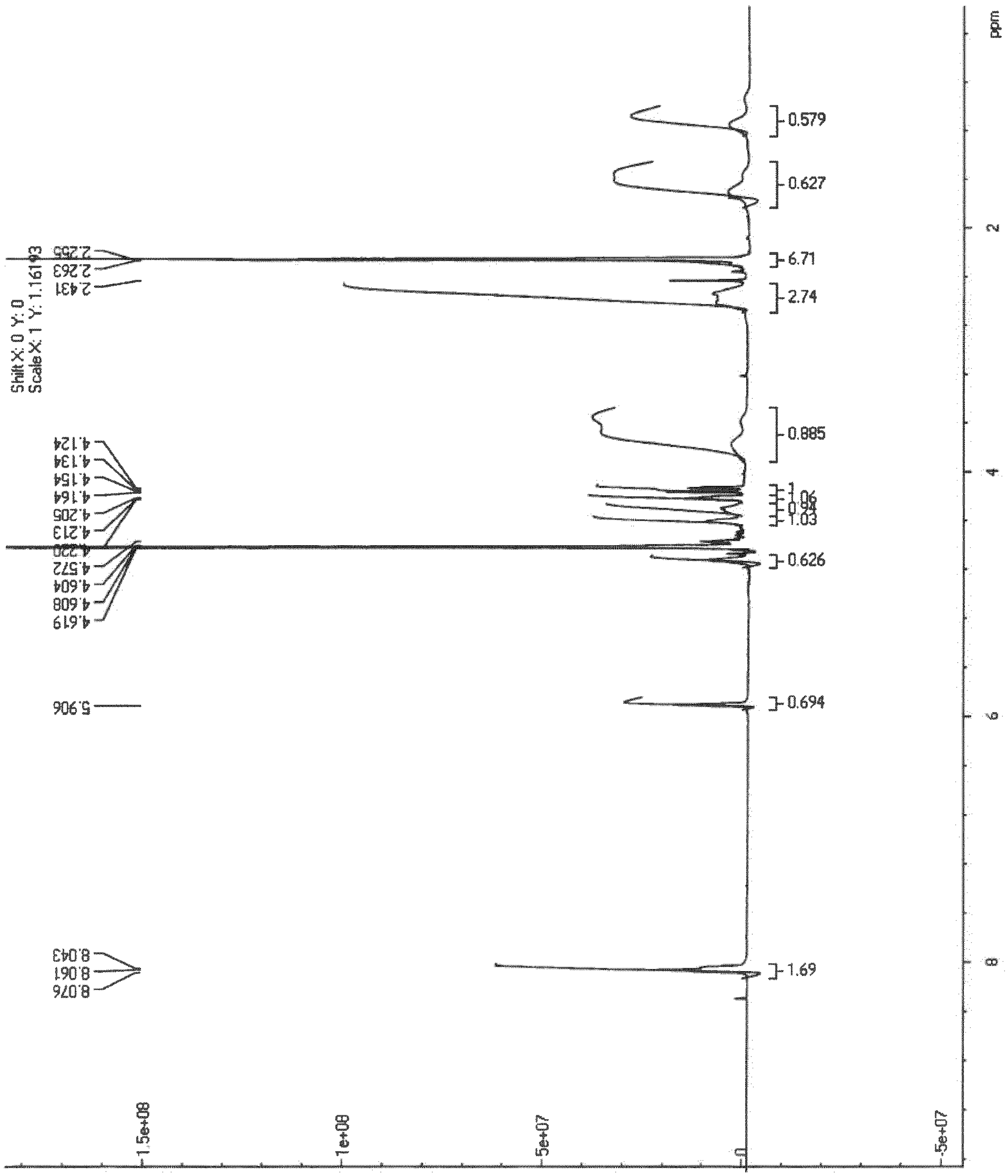
Shift X: 0 Y: 0
Scale X: 1 Y: 1.16193
2.255
2.263
2.431
4.124
4.134
4.154
4.164
4.205
4.213
4.220
4.572
4.604
4.608
4.619
5.906
8.043
8.061
8.076
0.579
0.627
6.71
2.74
0.885
1
1.06
0.94
1.03
0.626
0.694
1.69
ppm
2
4
6
8
1.5e+08
1e+08
5e+07
0
-5e+07

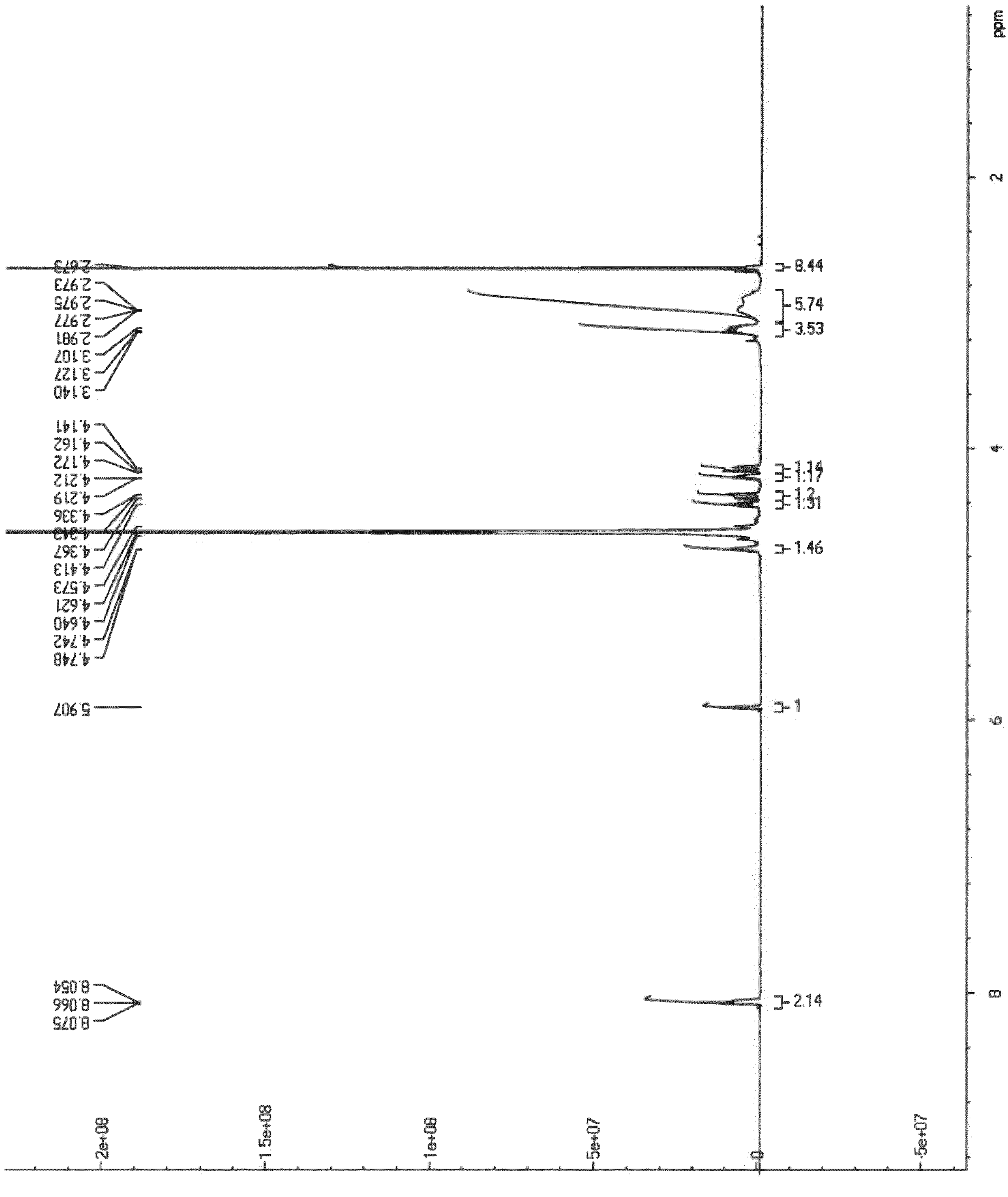
ppm
2
4
6
8
8.44
5.74
3.53
1.14
1.17
1.2
1.31
1.46
1
2.14
2e+08
1.5e+08
1e+08
5e+07
0
-5e+07
2.973
2.975
2.977
2.981
3.107
3.127
3.140
4.141
4.162
4.172
4.212
4.219
4.336
4.367
4.413
4.573
4.621
4.640
4.742
4.748
5.907
8.054
8.066
8.075

Fig. 3

Fig. 4

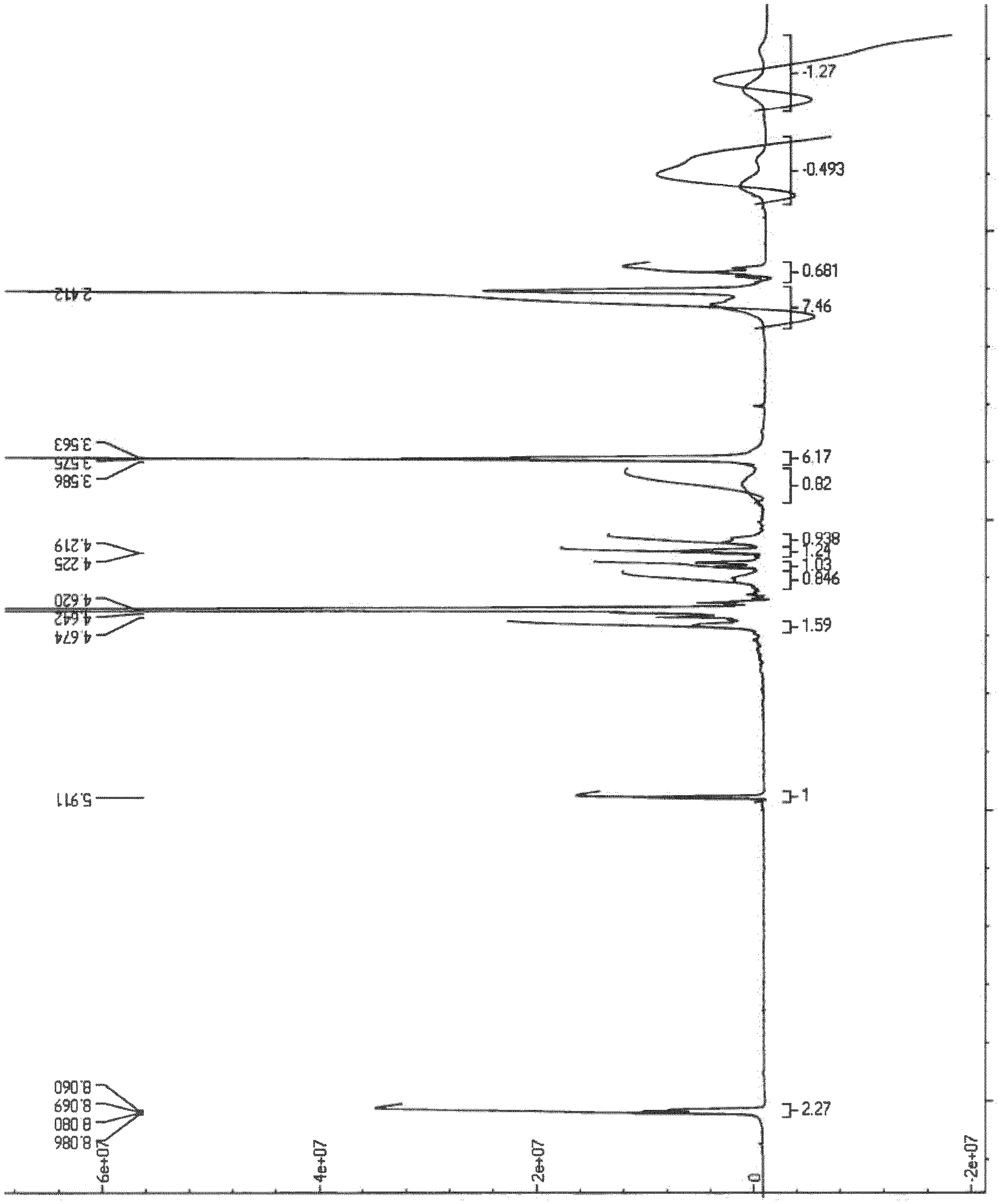
2.412
3.563
3.575
3.586
4.219
4.225
4.620
4.642
4.674
5.911
8.060
8.069
8.080
8.086
-1.27
-0.493
0.681
7.46
6.17
0.82
0.938
1.24
1.03
0.846
1.59
1
2.27
ppm
2
4
6
8
6e+07
4e+07
2e+07
0
-2e+07

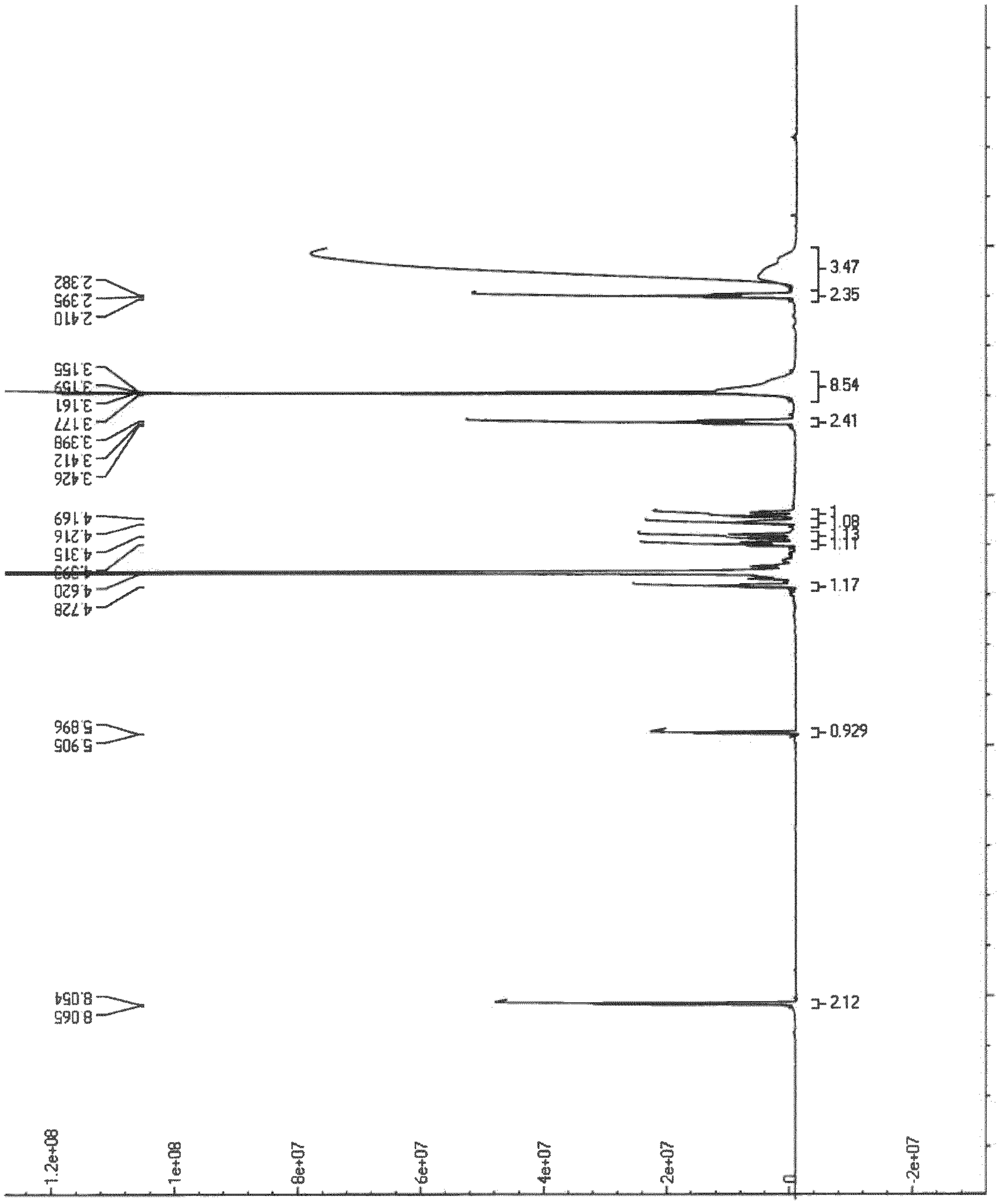
2.382
2.395
2.410
3.155
3.159
3.161
3.177
3.398
3.412
3.426
4.169
4.216
4.315
4.620
4.728
5.896
5.905
8.054
8.065
3.47
2.35
8.54
2.41
1.08
1.13
1.11
1.17
0.929
2.12
ppm
2
4
6
8
1.2e+08
1e+08
8e+07
6e+07
4e+07
2e+07
0
-2e+07

*Fig. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004022572 A
- EP 0269574 A
- WO 2003104482 A
- WO 1996040705 A
- WO 2001040245 A
- WO 2005117882 A
- WO 2004022572 A1
- WO 03104482 A1
- WO 9640705 A1
- WO 0140245 A1
- WO 2005117882 A2
- EP 0269574 A2

### Non-patent literature cited in the description

- **PALLE et al.** *Bioorganic & Medicinal Chemistry Letters,* 2004, vol. 14, 535-539
- **COTTAM et al.** *Journal of Medicinal Chemistry,* 1993, vol. 36 (22
- **PALLE et al.** *Bioorganic & Medicinal Chemistry Letters,* 2004, vol. 14 (2), 535-539